# EUROPEAN PATENT SPECIFICATION

(11) **EP 4 515 248 B1**
(45) Date of publication and mention of the grant of the patent: **26.08.2026**
(21) Application number: 23724516.2
(22) Date of filing: 21.04.2023
(51) Int. Cl.: G01N 33/96

(54) **METHODS FOR SAMPLE QUALITY ASSESSMENT**
VERFAHREN ZUR BEURTEILUNG DER PROBENQUALITÄT
PROCÉDÉS D'ÉVALUATION DE LA QUALITÉ D'UN ÉCHANTILLON

(30) Priority: 24.04.2022 US 202263334153 P
(43) Date of publication of application: 05.03.2025
(73) Proprietor: SomaLogic Operating Co., Inc., Boulder, CO 80301 (US)
(72) Inventor: ASTLING, David, Boulder, Colorado 80301 (US); WESTACOTT, Matthew, Boulder, Colorado 80301 (US); DROLET, Daniel, Boulder, Colorado 80301 (US)
(74) Representative: Mewburn Ellis LLP
(86) International application number: PCT/US2023/019358
(87) International publication number: WO 2023/211771

(56) References cited:
- WO-A1-2020/039085
- WO-A1-2020/092211
- CN-A- 111 474 371
- US-A1- 2013 103 321
- JANSEN EUGÈNE ET AL: "Quality control data of physiological and immunological biomarkers measured in serum and plasma", vol. 151, 1 November 2015 (2015-11-01), CH, pages 54 - 59, XP093053748, ISSN: 0047-6374, Retrieved from the Internet <URL:https://www.sciencedirect.com/science/article/pii/S0047637415300014/pdfft?md5=ff07e31cca58cbbf9cf83125bdf953db&pid=1-s2.0-S0047637415300014-main.pdf> DOI: 10.1016/j.mad.2015.06.004
- DANIELS JACLYN R. ET AL: "Stability of the Human Plasma Proteome to Pre-analytical Variability as Assessed by an Aptamer-Based Approach", JOURNAL OF PROTEOME RESEARCH, vol. 18, no. 10, 23 August 2019 (2019-08-23), pages 3661 - 3670, XP093040541, ISSN: 1535-3893, DOI: 10.1021/acs.jproteome.9b00320
- CUHADAR SERAP ET AL: "The effect of storage time and freeze-thaw cycles on the stability of serum samples", vol. 23, 1 January 2013 (2013-01-01), pages 70 - 77, XP093054234, Retrieved from the Internet <URL:https://www.ncbi.nlm.nih.gov/pmc/articles/PMC3900085/pdf/biochem_med-23-1-70-8.pdf> DOI: 10.11613/BM.2013.009
- CIÊNCIAS ET AL: "Identification of biomarkers for quality control in human biological serum samples", DOCTORAL THESIS, 1 January 2014 (2014-01-01), XP093053743, Retrieved from the Internet <URL:https://ubibliorum.ubi.pt/bitstream/10400.6/6175/1/3926_7654.pdf>
- SALEH AYACHE ET AL: "Effects of Storage Time and Exogenous Protease Inhibitors on Plasma Protein Levels", AMERICAN JOURNAL OF CLINICAL PATHOLOGY, vol. 126, no. 2, 1 August 2006 (2006-08-01), US, pages 174 - 184, XP055496720, ISSN: 0002-9173, DOI: 10.1309/3WM7XJ7RD8BCLNKX

## Description

### FIELD OF THE INVENTION

The present application relates generally to methods of assessing quality of a sample using biomarkers.

### BACKGROUND

Blood contains various cellular and humoral systems for reacting to injury or foreign and infectious agents. Small challenges can induce the innate immune system (complement system and cells such as macrophages) to release signals and enzymes, lead to activation of the platelets and trigger the coagulation of the blood. These signals are of interest because they can be directly involved in defense and repair systems and serve as markers for disease. However, such process signals may also be responsive to the effects of nutrients and ingredients in the food and beverages consumed that are absorbed into the bloodstream and could impact factors measured by certain tests. Thus, altering the duration of fasting before obtaining a blood sample can alter the apparent composition of serum or plasma such that physiologic information is masked by the pre-analytic variability imparted on the sample by the nutrients and ingredients in the food and beverages. The susceptibility of these processes and proteins to subtle alterations in fasting duration can compromise the use of them as biomarkers.

Currently, researchers in multivariate biology are concerned about pre-analytical sample variation (often called "batch effects"). The extent to which sample quality can be determined is largely limited to reliance on the subject to confirm the duration of fasting. These relatively crude methods limit the reliability of all but the hardiest and most robust protein measurements.

Specific techniques to determine compliance with fasting protocols are needed to monitor compliance, reject low quality samples, and/or make corrections in analytes of interest. Such techniques would improve evaluation of the quality of human or animal blood samples used in biomarker research, clinical diagnostic applications, bio-banks, and drug development.

Jansen et al., Mechanisms of Ageing and Development, 2015: reports quality control procedures and outcomes for 37 blood biomarkers measured over a four-year period in the MARK-AGE project. Cuhadar et al., Biochemia Medica, 2013: examines the analytical stability of 17 common clinical chemistry analytes in serum after storage at -20 °C for up to three months and after up to ten freeze-thaw cycles. Daniels et al., Journal of Proteome Research, 2019: evaluates how predefined pre-analytical variables in blood processing and plasma storage affect measured abundance of 1,305 plasma proteins using an aptamer-based assay.

### SUMMARY OF THE INVENTION

The invention is defined as set out in the appended claims. The invention in its general sense is defined by independent claim 1. The method involves measuring FGFP1 as one of the biomarkers. Methods involving biomarkers not including FGFP1, reagents, devices, systems and kits are useful for understanding the invention but are not claimed as such.

The present application includes biomarkers, methods, reagents, devices, systems, and kits for assessing sample quality. The biomarkers of the present application were identified using linear regression of measurements of a specific set of proteins affected by variation in a fasting time protocol. In some embodiments, the biomarker panels comprise proteins that are sensitive to fasting time.

In some embodiments, the methods comprise detecting the biomarkers using a multiplex slow off-rate aptamer-based assay described herein, for example, for assessing sample quality. In some embodiments not encompassed by the claimed invention, the sample is a blood sample, serum sample, or urine sample. In the claimed invention, the sample is a plasma sample.

In the claimed invention, the fasting time, optionally in hours, between a meal and a sample collection is predicted or estimated.

In some embodiments, methods of assessing quality of a sample collected from a subject are provided, the method comprising detecting the level of each of N biomarker proteins in the sample, wherein N is at least 2, and wherein one of the biomarker proteins is Fibroblast growth factor-binding protein 1 (FGFP1), and at least one of the N biomarker proteins is selected from Transmembrane emp24 domain-containing protein 10 (TMEDA), Glucagon, Gastric inhibitory polypeptide (GIP), Fatty acid-binding protein, liver (FABPL), Insulin, and Pancreatic hormone (PH), wherein the sample is a plasma sample.

In some embodiments, methods are provided comprising identifying the sample as an analysis sample or negative sample based on the level of the N biomarker proteins, wherein the analysis sample is a sample that is suitable for use in one or more of the following: protein biomarker discovery analysis, protein expression level analysis, a diagnostic method or a prognostic method, and the negative sample is a sample that is not suitable for use as an analysis sample.

In some embodiments, methods are provided a) contacting a plasma sample from a subject with a set of capture reagents, wherein each capture reagent of the set of capture reagents specifically binds to one biomarker protein being detected, and optionally b) measuring the level of each N biomarker protein with the set of capture reagents.

In some embodiments, methods are provided for comparing a plurality of samples collected from a plurality of subjects comprising detecting the level of each of N biomarker proteins in the sample, wherein N is at least 2, and wherein one of the biomarker proteins is Fibroblast growth factor-binding protein 1 (FGFP1), and at least one of the N biomarker proteins is selected from Transmembrane emp24 domain-containing protein 10 (TMEDA), Glucagon, Gastric inhibitory polypeptide (GIP), Fatty acid-binding protein, liver (FABPL), Insulin, and Pancreatic hormone (PH), wherein the sample is a plasma sample, and wherein the detecting is performed to determine the approximate fasting time that elapsed from a meal to sample collection. In some embodiments, the methods further comprise comparing the determined approximate times for each of the plurality of samples. In some embodiments, the determining is based on comparing the detected levels of each of the N biomarker proteins to reference levels, wherein the reference levels are average levels of the N biomarker proteins present in samples having fasting times of 0.5, 1, 3 and 12 hours.

In some embodiments, the detected levels of each of the N biomarker proteins compared to the reference levels indicates that the approximate fasting time that elapsed from meal to sample collection was greater than 0.5, greater than 1, greater than 1, greater than 3, greater than 4, greater than 5, greater than 6, greater than 7, greater than 8, greater than 9, greater than 10, greater than 11 or greater than 12 hours; or wherein the levels of each of the N biomarker proteins used in a linear regression model predicts the approximate fasting time that elapsed from meal to sample collection was greater than 0.1, greater than 0.5, greater than 1, greater than 1, greater than 3, greater than 4, greater than 5, greater than 6, greater than 7, greater than 8, greater than 9, greater than 10, greater than 11 or greater than 12 hours. In some embodiments, the determining is based on a panel of N biomarker proteins having an R² value of at least 0.600, at least 0.650, at least 0.700, at least 0.750, at least 0.800, at least 0.850, at least 0.900, or at least 0.950. In some embodiments, the methods include performing protein biomarker discovery analysis, protein expression level analysis, a diagnostic method or a prognostic method on the plurality of samples. In some embodiments, the methods comprise modifying a panel of proteins in the protein biomarker discovery analysis, the protein expression level analysis, the diagnostic method or the prognostic method based on the determined approximate time for each of the plurality of samples; or identifying one or more proteins in the sample as being affected by the approximate fasting time that elapsed from meal to sample collection; or identifying the level of one or more proteins in the sample as being affected by the approximate fasting time that elapsed from meal to sample collection; or changing the proteins used in a diagnostic, a prognostic or a health assessment related test based on the predicted approximate fasting time that elapsed from meal to sample collection; removing the proteins used in a diagnostic, a prognostic or a health assessment related test based on the predicted approximate fasting time that elapsed from meal to sample collection. In some embodiments, the panel of biomarker proteins is reduced in number of biomarker proteins measured.

In some embodiments, the determining measures compliance with a clinical trial sample collection and processing protocol. In some embodiments, the plurality of samples are collected at more than one sample collection site. In some embodiments, the plurality of samples from a first sample collection site are compared to a second plurality of samples from a second sample collection site. In some embodiments, one or more of the plurality of samples may be removed based on the fasting time.

In some embodiments, the determined approximate fasting time that elapsed from meal to sample collection greater than 0.5, greater than 1, greater than 1, greater than 3, greater than 4, greater than 5, greater than 6, greater than 7, greater than 8, greater than 9, greater than 10, greater than 11 or greater than 12 hours. In some embodiments, the determined approximate time is derived from the input of the level of each of N biomarker proteins in a statistical model. In some embodiments, the statistical model is a linear regression model.

In some embodiments the method further comprise modifying a panel of proteins in a protein biomarker discovery analysis, a protein expression level analysis, a diagnostic method or a prognostic method; identifying one or more proteins in the sample as being affected; identifying the level of one or more proteins in the sample as being affected; changing the proteins used in a diagnostic, a prognostic or a health assessment related test; or removing one or more proteins used in a diagnostic, a prognostic or a health assessment related test, each based on the outcome of the linear regression model.

In some embodiments, the sample was frozen and thawed prior to the detecting.

In any of the foregoing amendments, N is 8, N is 9, N is 10, N is 11, N is 12, N is 13, N is 14, N is 15, N is 16, N is 17, N is 18, N is 19, or N is 20. In some embodiments, N is 2, N is 3, N is 4, N is 5, N is 6, or N is 7. In some embodiments, all of the N biomarker proteins are selected from the list provided in Table 1.

In some embodiments, the subject is a human subject. In some embodiments, the sample is a plasma sample obtained from a whole blood sample. The method is performed *in vitro.* In some embodiments, the approximate duration of fasting time is derived from the input of the level of each of N biomarker proteins in a statistical model. In some embodiments, the statistical model is a linear regression model.

In some embodiments, the sample is identified as passing a quality assessment or failing a quality assessment. In some such embodiments, the identifying is based, at least in part, on the detected levels of the N biomarker proteins in the sample. In some embodiments, the identifying is based, at least in part, of the determined approximate fasting time. In some embodiments, the sample undergoes further analysis if it is identified as passing quality assessment, or the sample is discarded if it is identified as failing the quality assessment.

In some embodiments, the method comprises contacting biomarker proteins of the sample from the subject with a set of capture reagents, wherein each capture reagent of the set of capture reagents specifically binds to one biomarker protein being detected. In some embodiments, the method comprises contacting biomarker proteins of the sample from the subject with a set of capture reagents, wherein each capture reagent of the set of capture reagents specifically binds to a different biomarker protein being detected. In some embodiments, each capture reagent is an antibody or an aptamer. In some embodiments, each biomarker capture reagent is an aptamer. In some embodiments, at least one aptamer is a slow off-rate aptamer. In some embodiments, at least one slow off-rate aptamer comprises at least 1, at least 2, at least 3, at least 4, at least 5, at least 6, at least 7, at least 8, at least 9, or at least 10 nucleotides with modifications. In some embodiments, each slow off-rate aptamer binds to its target protein with an off rate (t½) of ≥ 30 minutes, ≥ 60 minutes, ≥ 90 minutes, ≥ 120 minutes, ≥ 150 minutes, ≥ 180 minutes, ≥ 210 minutes, or ≥ 240 minutes.

In some embodiments not encompassed by the claims, a kit is provided, the kit comprising N biomarker protein capture reagents, wherein N is at least 2 and at least 2 of the capture reagents bind to FGFP1, TMEDA, Glucagon, GIP, FABPL, Insulin, and PH. In some embodiments, N is 2, N is 3, N is 4, N is 5, N is 6, N is 7, N is 8, N is 9, N is 10, N is 11, N is 12, N is 13, N is 14, N is 15, N is 16, N is 17, N is 18, N is 19, or N is 20. In some embodiments, the kit comprises capture reagents from multiple fasting or sample processing panels.

In some embodiments, each of the N biomarker protein capture reagents specifically binds to a biomarker protein selected from Table 1. In some embodiments, each of the N biomarker capture reagents is an antibody or an aptamer. In some embodiments, each biomarker capture reagent is an aptamer. In some embodiments, at least one aptamer is a slow off-rate aptamer. In some embodiments, at least one slow off-rate aptamer comprises at least 1, at least 2, at least 3, at least 4, at least 5, at least 6, at least 7, at least 8, at least 9, or at least 10 nucleotides with modifications. In some embodiments, each slow off-rate aptamer binds to its target protein with an off rate (t½) of ≥ 30 minutes, ≥ 60 minutes, ≥ 90 minutes, ≥ 120 minutes, ≥ 150 minutes, ≥ 180 minutes, ≥ 210 minutes, or ≥ 240 minutes. In some embodiments not encompassed by the claims, the kit is for use in detecting the N biomarker proteins in a sample from a subject. In some embodiments not encompassed by the claims, the kit is for use in assessing the quality of the sample or a plurality of samples.

### BRIEF DESCRIPTION OF THE DRAWINGS

Fig. 1 illustrates a nonlimiting exemplary computer system for use with various computer-implemented methods described herein.

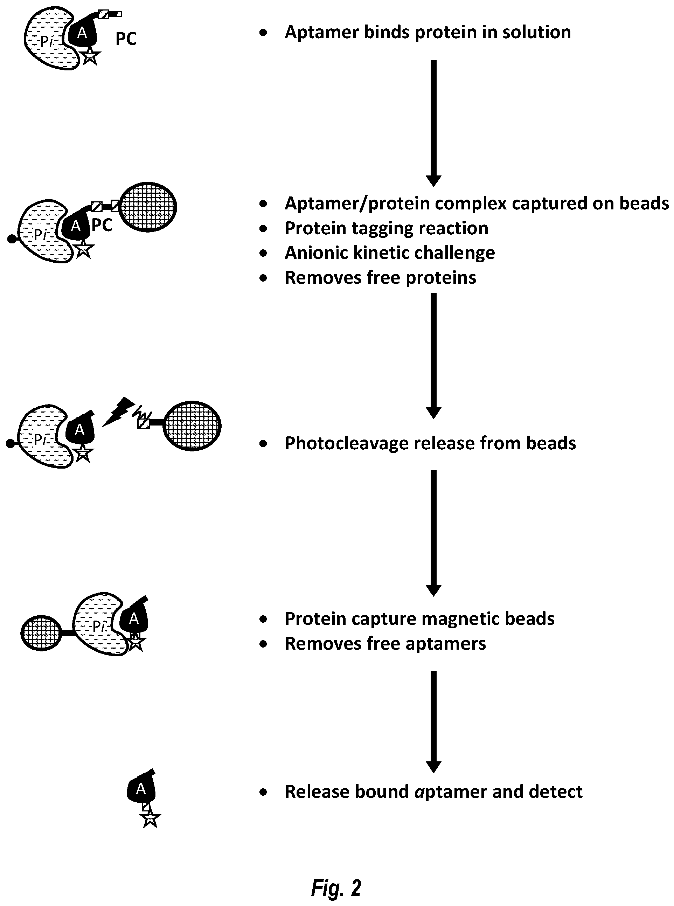

Fig. 2 illustrates a nonlimiting exemplary aptamer assay that can be used to detect one or more biomarkers in a biological sample.
Figs. 3-5 show certain exemplary modified pyrimidines that may be incorporated into aptamers, such as slow off-rate aptamers.

### DETAILED DESCRIPTION

Unless defined otherwise, technical and scientific terms used herein have the meaning commonly understood by one of ordinary skill in the art to which this invention belongs. Although any methods, devices, and materials similar or equivalent to those described herein can be used in the practice of the invention, certain methods, devices, and materials are described herein.

As used herein, the terms "comprises," "comprising," "includes," "including," "contains," "containing," and any variations thereof, are intended to cover a non-exclusive inclusion, such that a process, method, product-by-process, or composition of matter that comprises, includes, or contains an element or list of elements may include other elements not expressly listed.

"Biological sample", "sample", and "test sample" are used interchangeably herein to refer to any material, biological fluid, tissue, or cell obtained or otherwise derived from an individual. This includes blood (including whole blood, leukocytes, peripheral blood mononuclear cells, buffy coat, plasma, and serum), sputum, tears, mucus, nasal washes, nasal aspirate, urine, saliva, peritoneal washings, ascites, cystic fluid, glandular fluid, lymph fluid, bronchial aspirate, synovial fluid, joint aspirate, organ secretions, cells, a cellular extract, and cerebrospinal fluid. This also includes experimentally separated fractions of all of the preceding. For example, a blood sample can be fractionated into serum, plasma, or into fractions containing particular types of blood cells, such as red blood cells or white blood cells (leukocytes). In the claimed invention, a sample is a plasma sample. As used herein, a "plasma sample" comprises plasma and, optionally, one or more preservatives or additives. A plasma sample is isolated from whole blood and therefore does not comprise a substantial amount of any other blood component. In some embodiments not encompassed by the claims, a sample is a serum sample. As used herein, a "serum sample" comprises serum and, optionally, one or more preservatives or additives. A serum sample is isolated from whole blood and therefore does not comprise a substantial amount of any other blood component. In some embodiments not encompassed by the claims, a sample is a urine sample. As used herein, a "urine sample" comprises urine and, optionally, one or more preservatives or additives. In some embodiments, a blood sample is a dried blood spot. In some embodiments, a plasma sample is a dried plasma spot. In some embodiments, a sample can be a combination of samples from an individual, such as a combination of a tissue and fluid sample. The term "biological sample" also includes materials containing homogenized solid material, such as from a stool sample, a tissue sample, or a tissue biopsy, for example. The term "biological sample" also includes materials derived from a tissue culture or a cell culture. Any suitable methods for obtaining a biological sample can be employed; exemplary methods include, e.g., phlebotomy, swab (e.g., buccal swab), and a fine needle aspirate biopsy procedure. Exemplary tissues susceptible to fine needle aspiration include lymph node, lung, thyroid, breast, pancreas, and liver. Samples can also be collected, e.g., by micro dissection (e.g., laser capture micro dissection (LCM) or laser micro dissection (LMD)), bladder wash, smear (e.g., a PAP smear), or ductal lavage. A "biological sample" obtained or derived from an individual includes any such sample that has been processed in any suitable manner after being obtained from the individual.

Further, in some embodiments, a biological sample may be derived by taking biological samples from a number of individuals and pooling them, or pooling an aliquot of each individual's biological sample. The pooled sample may be treated as described herein for a sample from a single individual, and, for example, if a poor sample quality is established in the pooled sample, then each individual biological sample can be re-tested to determine which must be discarded, or the entire group of samples may be discarded if known to have been handled or processed in the same way.

For purposes of this specification, the phrase "data attributed to a biological sample from an individual" is intended to mean that the data in some form derived from, or were generated using, the biological sample of the individual. The data may have been reformatted, revised, or mathematically altered to some degree after having been generated, such as by conversion from units in one measurement system to units in another measurement system; but, the data are understood to have been derived from, or were generated using, the biological sample.

"Target", "target molecule", and "analyte" are used interchangeably herein to refer to any molecule of interest that may be present in a biological sample. A "molecule of interest" includes any minor variation of a particular molecule, such as, in the case of a protein, for example, minor variations in amino acid sequence, disulfide bond formation, glycosylation, lipidation, acetylation, phosphorylation, or any other manipulation or modification, such as conjugation with a labeling component, which does not substantially alter the identity of the molecule. A "target molecule", "target", or "analyte" refers to a set of copies of one type or species of molecule or multi-molecular structure. Exemplary target molecules include proteins, polypeptides, nucleic acids, carbohydrates, lipids, polysaccharides, glycoproteins, hormones, receptors, antigens, antibodies, affybodies, antibody mimics, viruses, pathogens, toxic substances, substrates, metabolites, transition state analogs, cofactors, inhibitors, drugs, dyes, nutrients, growth factors, cells, tissues, and any fragment or portion of any of the foregoing. In some embodiments, a target molecule is a protein, in which case the target molecule may be referred to as a "target protein."

As used herein, a "capture agent' or "capture reagent" refers to a molecule that is capable of binding specifically to a biomarker. A "target protein capture reagent" refers to a molecule that is capable of binding specifically to a target protein. Nonlimiting exemplary capture reagents include aptamers, antibodies, adnectins, ankyrins, other antibody mimetics and other protein scaffolds, autoantibodies, chimeras, small molecules, nucleic acids, lectins, ligand-binding receptors, imprinted polymers, avimers, peptidomimetics, hormone receptors, cytokine receptors, synthetic receptors, and modifications and fragments of any of the aforementioned capture reagents. In some embodiments, a capture reagent is selected from an aptamer and an antibody.

The term "antibody" refers to full-length antibodies of any species and fragments and derivatives of such antibodies, including Fab fragments, F(ab')₂ fragments, single chain antibodies, Fv fragments, and single chain Fv fragments. The term "antibody" also refers to synthetically-derived antibodies, such as phage display-derived antibodies and fragments, affybodies, nanobodies, etc.

As used herein, "marker" and "biomarker" are used interchangeably to refer to a target molecule that indicates or is a sign of a normal or abnormal process in an individual or of a high or low quality sample. More specifically, a "marker" or "biomarker" is an anatomic, physiologic, biochemical, or molecular parameter associated with the presence of a specific state or process. Biomarkers are detectable and measurable by a variety of methods including laboratory assays and medical imaging.

As used herein, "biomarker level" and "level" refer to a measurement that is made using any analytical method for detecting the biomarker in a biological sample and that indicates the presence, absence, absolute amount or concentration, relative amount or concentration, titer, a level, an expression level, a ratio of measured levels, or the like, of, for, or corresponding to the biomarker in the biological sample. The exact nature of the "level" depends on the specific design and components of the particular analytical method employed to detect the biomarker.

When a biomarker indicates or is a sign of a poor quality sample, that biomarker is generally described as being either over-expressed or under-expressed as compared to an expression level or value of the biomarker that indicates or is a sign of a normal or quality sample. "Up-regulation", "up-regulated", "over-expression", "over-expressed", and any variations thereof are used interchangeably to refer to a value or level of a biomarker in a biological sample that is greater than a value or level (or range of values or levels) of the biomarker that is typically detected in similar, properly handled biological samples.

"Down-regulation", "down-regulated", "under-expression", "under-expressed", and any variations thereof are used interchangeably to refer to a value or level of a biomarker in a biological sample that is less than a value or level (or range of values or levels) of the biomarker that is typically detected in similar, properly handled biological samples.

Further, a biomarker that is either over-expressed or under-expressed can also be referred to as being "differentially expressed" or as having a "differential level" or "differential value" as compared to a "normal" expression level or value of the biomarker that indicates or is a sign of a normal process or proper sample handling. Thus, "differential expression" of a biomarker can also be referred to as a variation from a "normal" expression level of the biomarker.

A "control level" of a target molecule refers to the level of the target molecule in a fasted sample of the same sample type. Control level may refer to the average level of the target molecule in fasted samples from a population of individuals.

As used herein, "individual," "subject," and "patient" are used interchangeably to refer to a mammal. A mammalian individual can be a human or non-human. In various embodiments, the individual is a human. A healthy or normal individual is an individual in which the disease or condition of interest (including, for example, chronic heart failure and cardiovascular events such as myocardial infarction, stroke and hospitalization for heart failure) is not detectable by conventional diagnostic methods.

As used herein, "detecting" or "determining" with respect to a biomarker value includes the use of both the instrument used to observe and record a signal corresponding to a biomarker level and the material/s required to generate that signal. In various embodiments, the biomarker level is detected using any suitable method, including fluorescence, chemiluminescence, surface plasmon resonance, surface acoustic waves, mass spectrometry, infrared spectroscopy, Raman spectroscopy, atomic force microscopy, scanning tunneling microscopy, electrochemical detection methods, nuclear magnetic resonance, quantum dots, and the like.

As used herein, "sample processing" and "sample handling" refer to steps or procedures performed on a sample, such as a blood sample, after sample collection in order to prepare it for storage or analysis. In some embodiments, sample processing steps include centrifugation of the sample, decanting or aspirating the supernatant and freezing.

As used herein, "time-to-centrifugation" means the time elapsed between the moment that a blood sample is collected from a subject into a tube to the moment that the tube begins spinning in a centrifuge. In some embodiments, time-to-centrifugation is measured in hours. In some embodiments, ideal time-to-centrifugation for optimal sample quality is two hours or less. In some embodiments, the time-to-centrifugation is rounded to the nearest hour. In some embodiments, the time-to-centrifugation is rounded to the nearest half hour. Thus, in some embodiments, a time-to-centrifugation of less than a half hour or less than a quarter of an hour is rounded to zero.

As used herein, "time-to-decant" means the time elapsed between the moment that centrifugation of a sample is complete to the moment that the supernatant of the centrifuged sample begins to be decanted or aspirated from the precipitate. In some embodiments, time-to-decant is measured in hours. In some embodiments, ideal time-to-decant for optimal sample quality is less than one hour or less than half an hour, or less than a quarter hour. In some embodiments, the time-to-decant is rounded to the nearest hour. In some embodiments, the time-to-decant is rounded to the nearest half hour. Thus, in some embodiments, a time-to-decant of less than a half hour or less than a quarter of an hour is rounded to zero.

As used herein, "time-to-freeze" means the time elapsed between the moment that decanting or aspirating of a centrifuged sample is complete to the moment that the decanted or aspirated sample is placed in conditions at or under -20°C. In some embodiments, time-to-freeze is measured in hours. In some embodiments, ideal time-to-decant for optimal sample quality is less than one hour or less than half an hour, or less than a quarter hour. In some embodiments, the time-to-freeze is rounded to the nearest hour. In some embodiments, the time-to-freeze is rounded to the nearest half hour. Thus, in some embodiments, a time-to-freeze of less than a half hour or less than a quarter of an hour is rounded to zero.

As used herein "fasting time" means the time elapsed between a meal, ingestion of liquids or both, and sample collection. In some embodiments the time is measured in hours. In some embodiments, the fasting time is 0.5 hours or more, 1 hour or more, 3 hours or more or 12 hours or more. During the fasting period, the subject preferably does not ingest any food or liquids with calories. In some embodiments, the subject does not ingest any liquids other than water during the fasting period. In other embodiments, the subject may ingest small amounts of low calorie beverages, such as tea, coffee, or diluted juices.

"Solid support" refers herein to any substrate having a surface to which molecules may be attached, directly or indirectly, through either covalent or non-covalent bonds. A "solid support" can have a variety of physical formats, which can include, for example, a membrane; a chip (e.g., a protein chip); a slide (e.g., a glass slide or coverslip); a column; a hollow, solid, semi-solid, pore- or cavity- containing particle, such as, for example, a bead; a gel; a fiber, including a fiber optic material; a matrix; and a sample receptacle. Exemplary sample receptacles include sample wells, tubes, capillaries, vials, and any other vessel, groove or indentation capable of holding a sample. A sample receptacle can be contained on a multi-sample platform, such as a microtiter plate, slide, microfluidics device, and the like. A support can be composed of a natural or synthetic material, an organic or inorganic material. The composition of the solid support on which capture reagents are attached generally depends on the method of attachment (e.g., covalent attachment). Other exemplary receptacles include microdroplets and microfluidic controlled or bulk oil/aqueous emulsions within which assays and related manipulations can occur. Suitable solid supports include, for example, plastics, resins, polysaccharides, silica or silica-based materials, functionalized glass, modified silicon, carbon, metals, inorganic glasses, membranes, nylon, natural fibers (such as, for example, silk, wool and cotton), polymers, and the like. The material composing the solid support can include reactive groups such as, for example, carboxy, amino, or hydroxyl groups, which are used for attachment of the capture reagents. Polymeric solid supports can include, e.g., polystyrene, polyethylene glycol tetraphthalate, polyvinyl acetate, polyvinyl chloride, polyvinyl pyrrolidone, polyacrylonitrile, polymethyl methacrylate, polytetrafluoroethylene, butyl rubber, styrenebutadiene rubber, natural rubber, polyethylene, polypropylene, (poly)tetrafluoroethylene, (poly)vinylidenefluoride, polycarbonate, and polymethylpentene. Suitable solid support particles that can be used include, e.g., encoded particles, such as Luminex^{®}-type encoded particles, magnetic particles, and glass particles.

### Exemplary Uses of Biomarkers

Methods are provided for evaluating or assessing sample quality by detecting one or more biomarker values corresponding to one or more biomarkers present in a sample from an individual, such as a blood, serum, or plasma sample, by any number of analytical methods, including any of the analytical methods described herein. These biomarkers are, for example, present at different levels in samples with differing quality. In some embodiments, the differing sample quality is due to differences in fasting times. Detection of the different levels of a biomarker in a sample can be used, for example, to estimate the fasting time that elapsed between a meal and sample collection. For the claimed invention, the sample is plasma, and the method is used to determine the approximate fasting time that elapsed from a meal to sample collection.

In addition to detecting biomarkers to assess sample quality, biomarkers can be used in diagnostic applications or to determine if a disease or condition is present in the subject from whom the sample was taken. In some embodiments, only samples that pass the quality assessment are further analyzed for diagnostic applications.

### Detection and Determination of Biomarkers and Biomarker Levels

Levels of the biomarkers described herein can be detected using any of a variety of known analytical methods. In one embodiment, a biomarker level is detected using a capture reagent. In various embodiments, the capture reagent can be exposed to the biomarker in solution or can be exposed to the biomarker while the capture reagent is immobilized on a solid support. In other embodiments, the capture reagent contains a feature that is reactive with a secondary feature on a solid support. In these embodiments, the capture reagent can be exposed to the biomarker in solution, and then the feature on the capture reagent can be used in conjunction with the secondary feature on the solid support to immobilize the biomarker on the solid support. The capture reagent is selected based on the type of analysis to be conducted. Capture reagents include but are not limited to aptamers, antibodies, adnectins, ankyrins, other antibody mimetics and other protein scaffolds, autoantibodies, chimeras, small molecules, F(ab')₂ fragments, single chain antibody fragments, Fv fragments, single chain Fv fragments, nucleic acids, lectins, ligand-binding receptors, affibodies, nanobodies, imprinted polymers, avimers, peptidomimetics, hormone receptors, cytokine receptors, and synthetic receptors, and modifications and fragments of these.

In some embodiments, a biomarker level is detected using a biomarker/capture reagent complex.

In some embodiments, the biomarker level is derived from the biomarker/capture reagent complex and is detected indirectly, such as, for example, as a result of a reaction that is subsequent to the biomarker/capture reagent interaction, but is dependent on the formation of the biomarker/capture reagent complex.

In some embodiments, the biomarker level is detected directly from the biomarker in a biological sample.

In some embodiments, biomarkers are detected using a multiplexed format that allows for the simultaneous detection of two or more biomarkers in a biological sample. In some embodiments of the multiplexed format, capture reagents are immobilized, directly or indirectly, covalently or non-covalently, in discrete locations on a solid support. In some embodiments, a multiplexed format uses discrete solid supports where each solid support has a unique capture reagent associated with that solid support, such as, for example quantum dots. In some embodiments, an individual device is used for the detection of each one of multiple biomarkers to be detected in a biological sample. Individual devices can be configured to permit each biomarker in the biological sample to be processed simultaneously. For example, a microtiter plate can be used such that each well in the plate is used to uniquely analyze one or more biomarkers to be detected in a biological sample.

In one or more of the foregoing embodiments, a fluorescent tag can be used to label a component of the biomarker/capture reagent complex to enable the detection of the biomarker level. In various embodiments, the fluorescent label can be conjugated to a capture reagent specific to any of the biomarkers described herein using known techniques, and the fluorescent label can then be used to detect the corresponding biomarker level. Suitable fluorescent labels include rare earth chelates, fluorescein and its derivatives, rhodamine and its derivatives, dansyl, allophycocyanin, PBXL-3, Qdot 605, Lissamine, phycoerythrin, Texas Red, and other such compounds.

In some embodiments, the fluorescent label is a fluorescent dye molecule. In some embodiments, the fluorescent dye molecule includes at least one substituted indolium ring system in which the substituent on the 3-carbon of the indolium ring contains a chemically reactive group or a conjugated substance. In some embodiments, the dye molecule includes an AlexFluor molecule, such as, for example, AlexaFluor 488, AlexaFluor 532, AlexaFluor 647, AlexaFluor 680, or AlexaFluor 700. In other embodiments, the dye molecule includes a first type and a second type of dye molecule, such as, e.g., two different AlexaFluor molecules. In some embodiments, the dye molecule includes a first type and a second type of dye molecule, and the two dye molecules have different emission spectra.

Fluorescence can be measured with a variety of instrumentation compatible with a wide range of assay formats. For example, spectrofluorimeters have been designed to analyze microtiter plates, microscope slides, printed arrays, cuvettes, etc. See Principles of Fluorescence Spectroscopy, by J.R. Lakowicz, Springer Science + Business Media, Inc., 2004. See Bioluminescence & Chemiluminescence: Progress & Current Applications; Philip E. Stanley and Larry J. Kricka editors, World Scientific Publishing Company, January 2002.

In one or more embodiments, a chemiluminescence tag can optionally be used to label a component of the biomarker/capture complex to enable the detection of a biomarker level. Suitable chemiluminescent materials include any of oxalyl chloride, Rodamin 6G, Ru(bipy)₃²⁺ , TMAE (tetrakis(dimethylamino)ethylene), Pyrogallol (1,2,3-trihydroxibenzene), Lucigenin, peroxyoxalates, Aryl oxalates, Acridinium esters, dioxetanes, and others.

In some embodiments, the detection method includes an enzyme/substrate combination that generates a detectable signal that corresponds to the biomarker level. Generally, the enzyme catalyzes a chemical alteration of the chromogenic substrate which can be measured using various techniques, including spectrophotometry, fluorescence, and chemiluminescence. Suitable enzymes include, for example, luciferases, luciferin, malate dehydrogenase, urease, horseradish peroxidase (HRPO), alkaline phosphatase, betagalactosidase, glucoamylase, lysozyme, glucose oxidase, galactose oxidase, and glucose-6-phosphate dehydrogenase, uricase, xanthine oxidase, lactoperoxidase, microperoxidase, and the like.

In some embodiments, the detection method can be a combination of fluorescence, chemiluminescence, radionuclide or enzyme/substrate combinations that generate a measurable signal. In some embodiments, multimodal signaling could have unique and advantageous characteristics in biomarker assay formats.

In some embodiments, the biomarker levels for the biomarkers described herein can be detected using any analytical methods including, singleplex aptamer assays, multiplexed aptamer assays, singleplex or multiplexed immunoassays, mRNA expression profiling, miRNA expression profiling, mass spectrometric analysis, histological/cytological methods, etc. as discussed below. For the claimed invention, the biomarkers measured are proteins (i.e. not mRNA).

### Determination of Biomarker Levels using Aptamer-Based Assays

Assays directed to the detection and quantification of biomarker molecules in biological samples and other samples are important tools in scientific research and in the health care field. One class of such assays involves the use of a microarray that includes one or more aptamers immobilized on a solid support. The aptamers are each capable of binding to a target molecule in a highly specific manner and with very high affinity. See, e.g., U.S. Patent No. 5,475,096 entitled "Nucleic Acid Ligands"; see also, e.g., U.S. Patent No. 6,242,246, U.S. Patent No. 6,458,543, and U.S. Patent No. 6,503,715, each of which is entitled "Nucleic Acid Ligand Diagnostic Biochip". Once the microarray is contacted with a sample, the aptamers bind to their respective target molecules present in the sample and thereby enable a determination of a biomarker level corresponding to a biomarker.

As used herein, an "aptamer" refers to a nucleic acid that has a specific binding affinity for a target molecule. It is recognized that affinity interactions are a matter of degree; however, in this context, the "specific binding affinity" of an aptamer for its target means that the aptamer binds to its target generally with a much higher degree of affinity than it binds to other components in a test sample. An "aptamer" is a set of copies of one type or species of nucleic acid molecule that comprises a particular nucleotide sequence. An aptamer can include any suitable number of nucleotides, including any number of chemically modified nucleotides. "Aptamers" refers to more than one such set of molecules. Different aptamers can have either the same or different numbers of nucleotides. Aptamers can be DNA or RNA or chemically modified nucleic acids and can be single-stranded, double-stranded, or contain double-stranded regions, and can include higher ordered structures. An aptamer can also be a photoaptamer, where a photoreactive or chemically reactive functional group is included in the aptamer to allow it to be covalently linked to its corresponding target. Any of the aptamer methods disclosed herein can include the use of two or more aptamers that specifically bind the same target molecule. As further described below, an aptamer may include a tag. If an aptamer includes a tag, all copies of the aptamer need not have the same tag. Moreover, if different aptamers each include a tag, these different aptamers can have either the same tag or a different tag.

An aptamer can be identified using any known method, including the SELEX process. Once identified, an aptamer can be prepared or synthesized in accordance with any known method, including chemical synthetic methods and enzymatic synthetic methods.

The terms "SELEX" and "SELEX process" are used interchangeably herein to refer generally to a combination of (1) the selection of aptamers that interact with a target molecule in a desirable manner, for example binding with high affinity to a protein, with (2) the amplification of those selected nucleic acids. The SELEX process can be used to identify aptamers with high affinity to a specific target or biomarker.

SELEX generally includes preparing a candidate mixture of nucleic acids, binding of the candidate mixture to the desired target molecule to form an affinity complex, separating the affinity complexes from the unbound candidate nucleic acids, separating and isolating the nucleic acid from the affinity complex, purifying the nucleic acid, and identifying a specific aptamer sequence. The process may include multiple rounds to further refine the affinity of the selected aptamer. The process can include amplification steps at one or more points in the process. See, e.g., U.S. Patent No. 5,475,096, entitled "Nucleic Acid Ligands". The SELEX process can be used to generate an aptamer that covalently binds its target as well as an aptamer that non-covalently binds its target. See, e.g., U.S. Patent No. 5,705,337 entitled "Systematic Evolution of Nucleic Acid Ligands by Exponential Enrichment: Chemi-SELEX."

The SELEX process can be used to identify high-affinity aptamers containing modified nucleotides that confer improved characteristics on the aptamer, such as, for example, improved *in vivo* stability or improved delivery characteristics. Examples of such modifications include chemical substitutions at the ribose and/or phosphate and/or base positions. SELEX process-identified aptamers containing modified nucleotides are described in U.S. Patent No. 5,660,985, entitled "High Affinity Nucleic Acid Ligands Containing Modified Nucleotides", which describes oligonucleotides containing nucleotide derivatives chemically modified at the 5'- and 2'-positions of pyrimidines. U.S. Patent No. 5,580,737, see supra, describes highly specific aptamers containing one or more nucleotides modified with 2'-amino (2'-NH2), 2'-fluoro (2'-F), and/or 2'-O-methyl (2'-Ome). See also, U.S. Patent Application Publication 20090098549, entitled "SELEX and PHOTOSELEX", which describes nucleic acid libraries having expanded physical and chemical properties and their use in SELEX and photoSELEX.

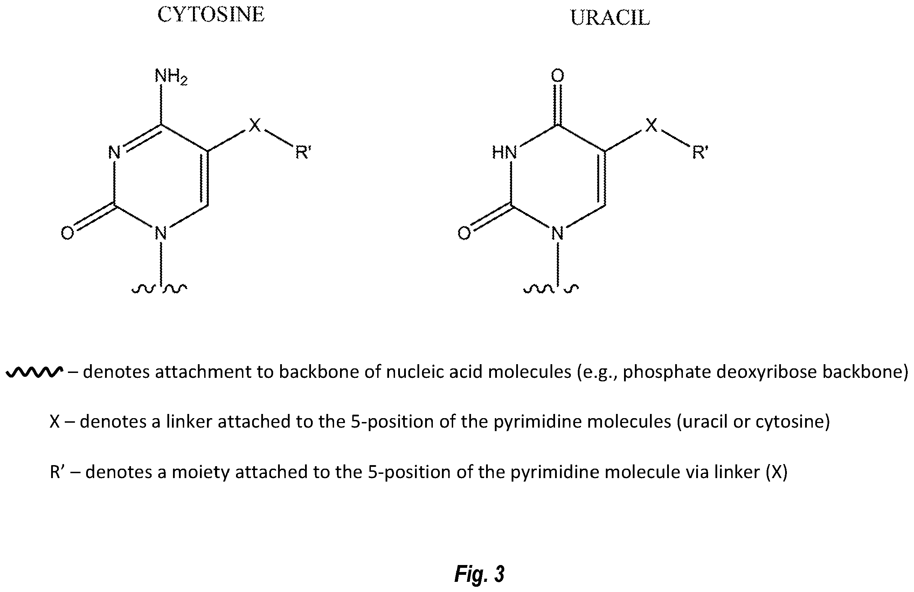

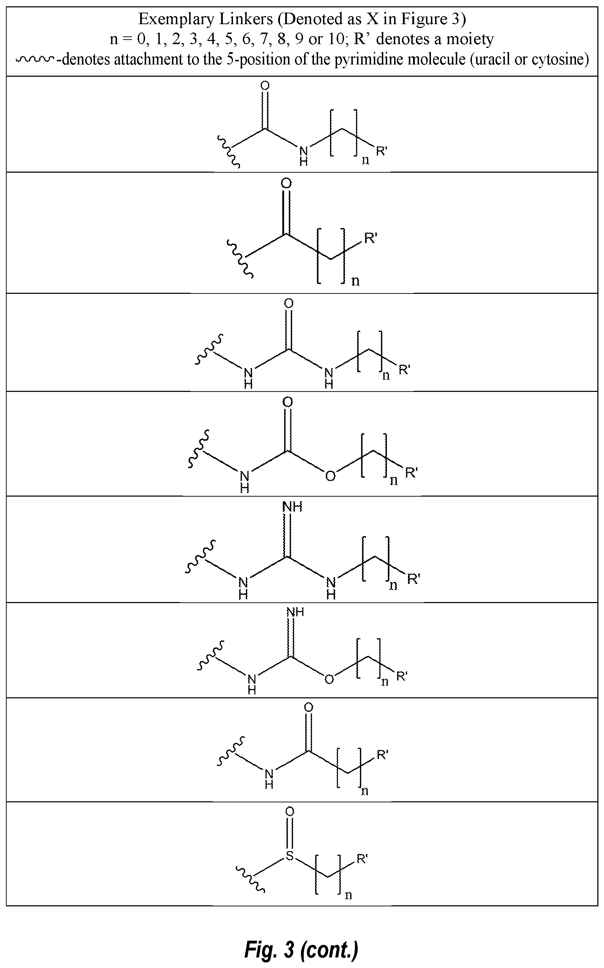

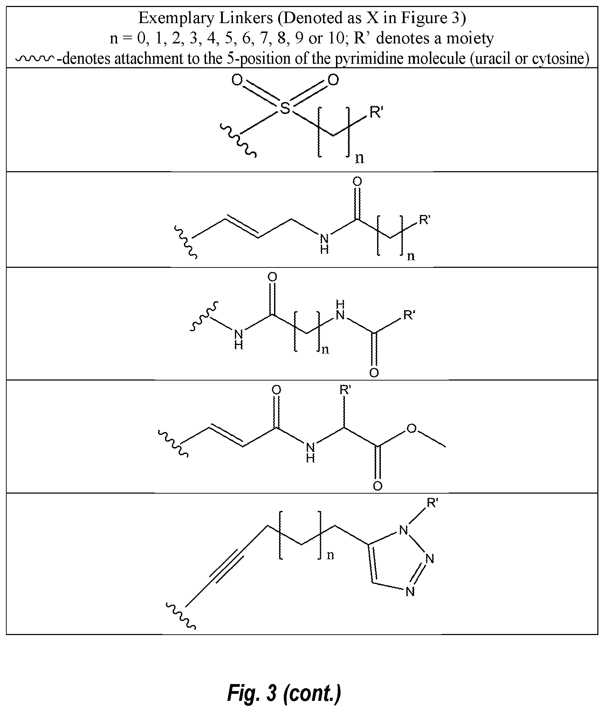

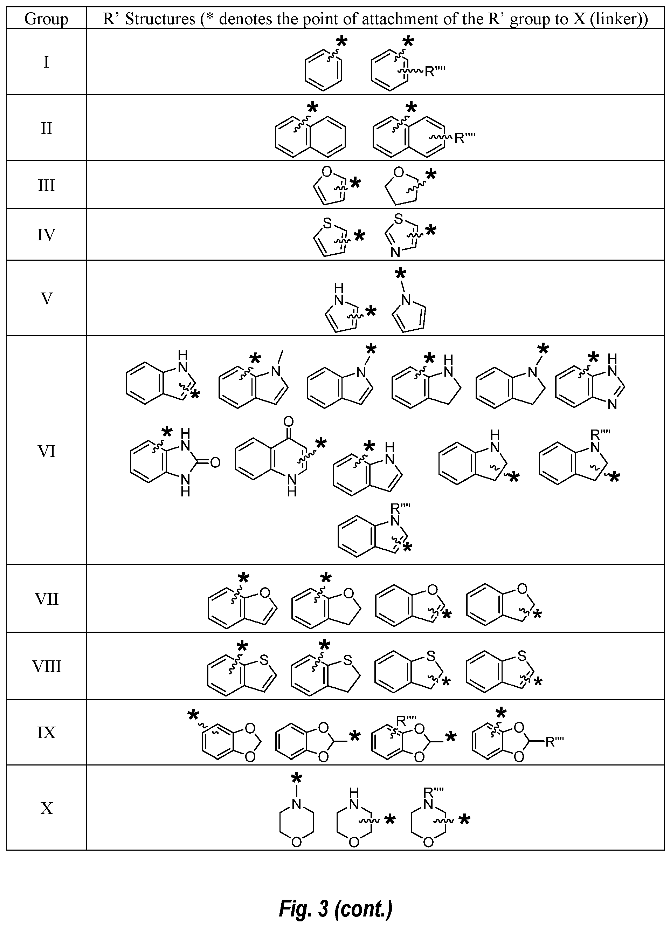

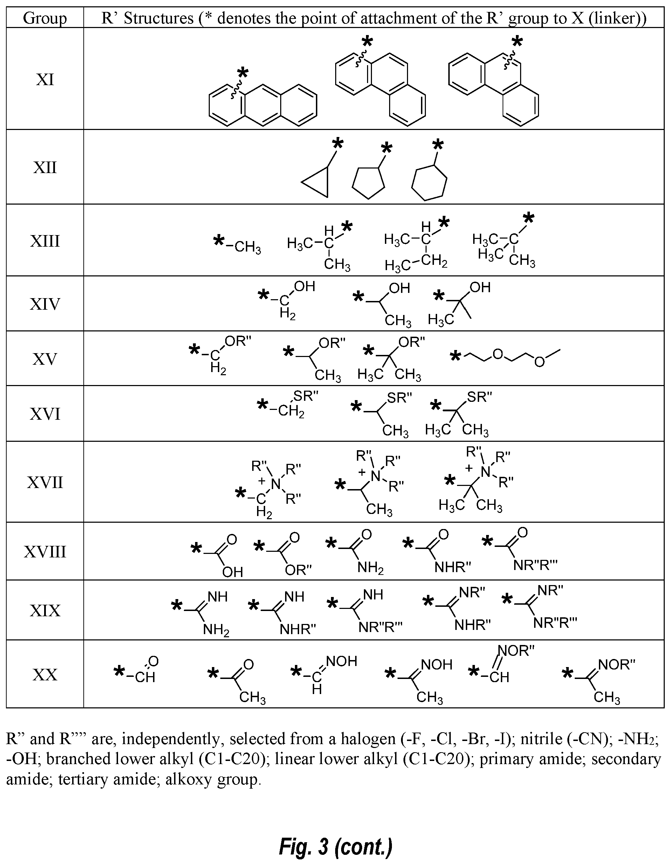

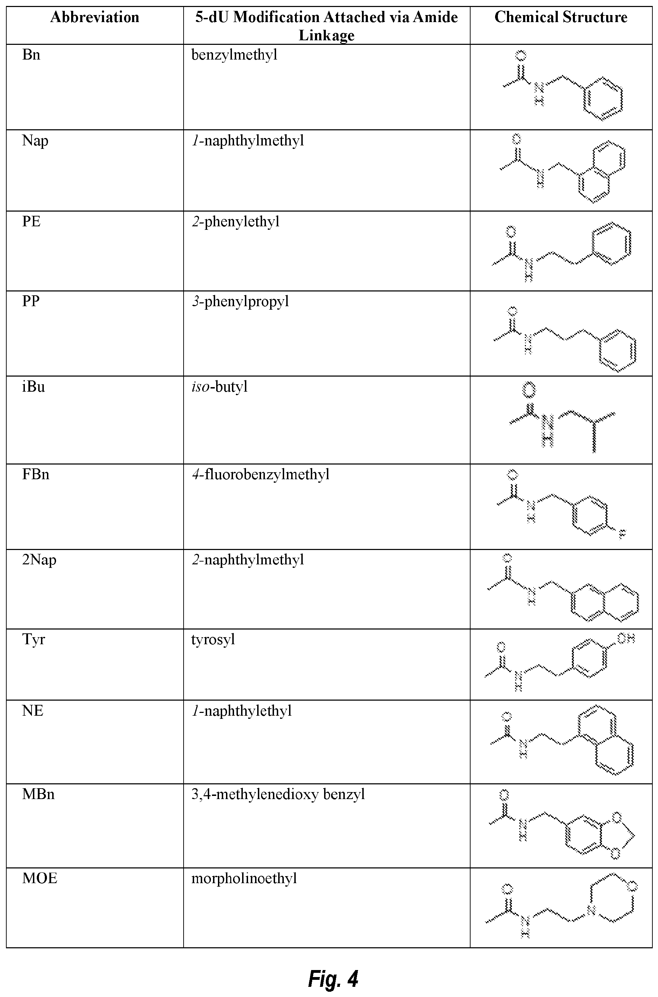

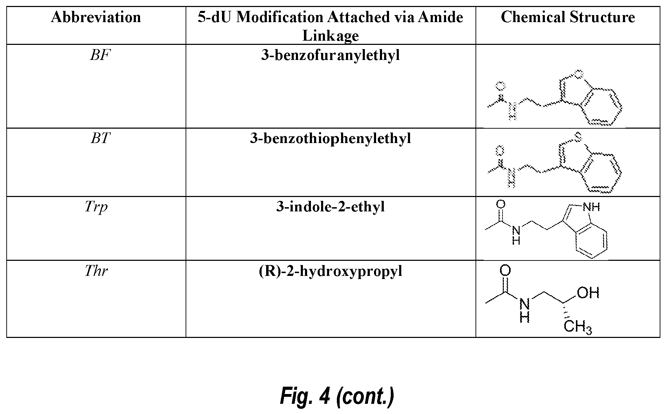

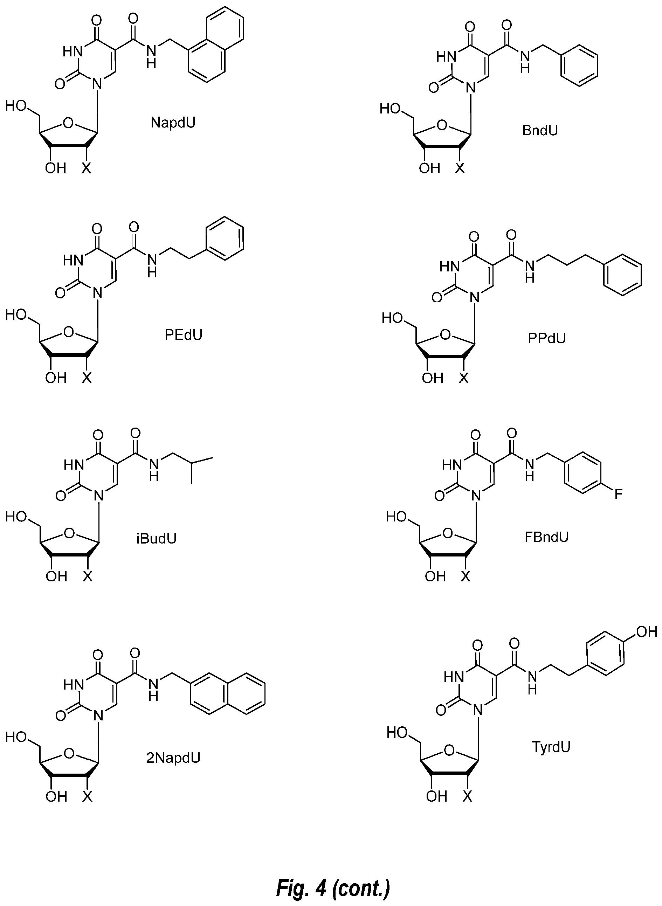

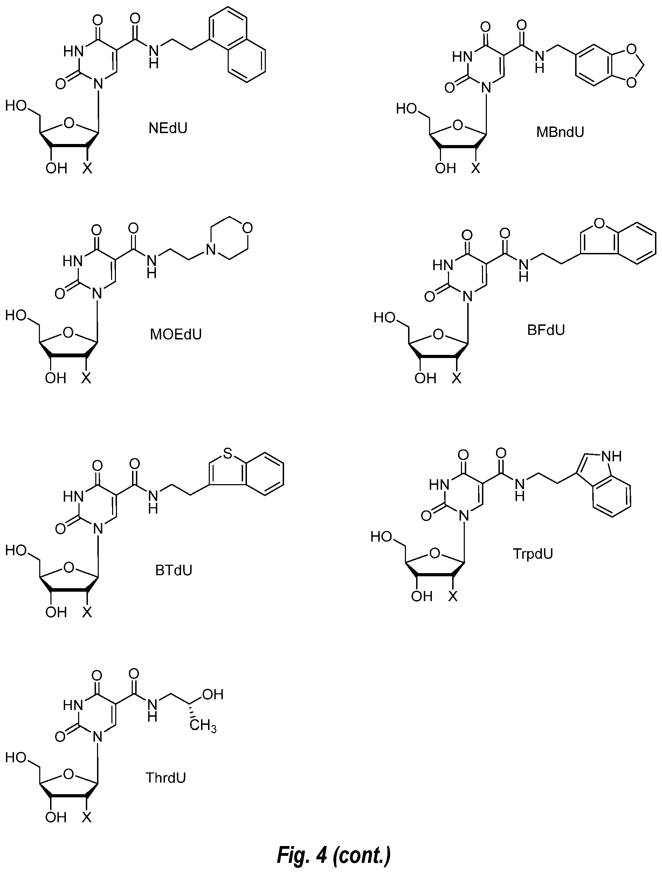

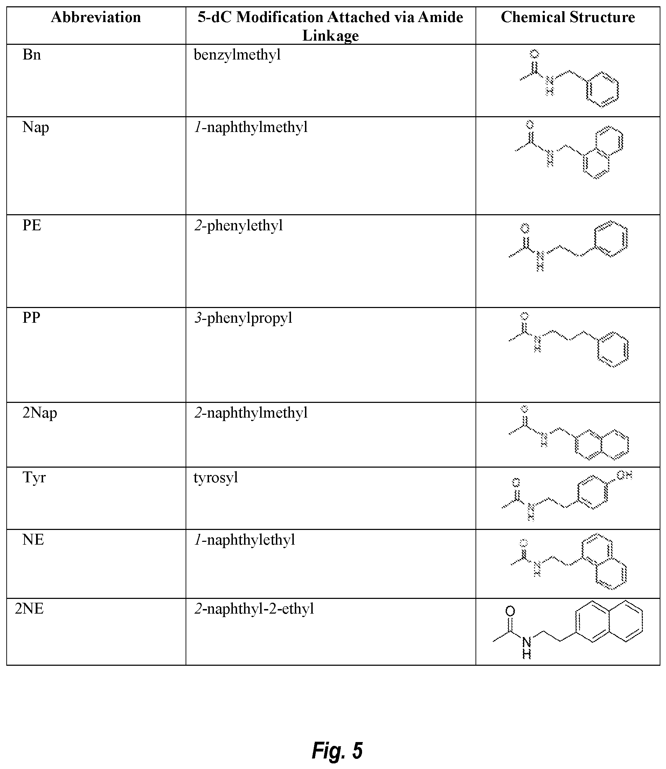

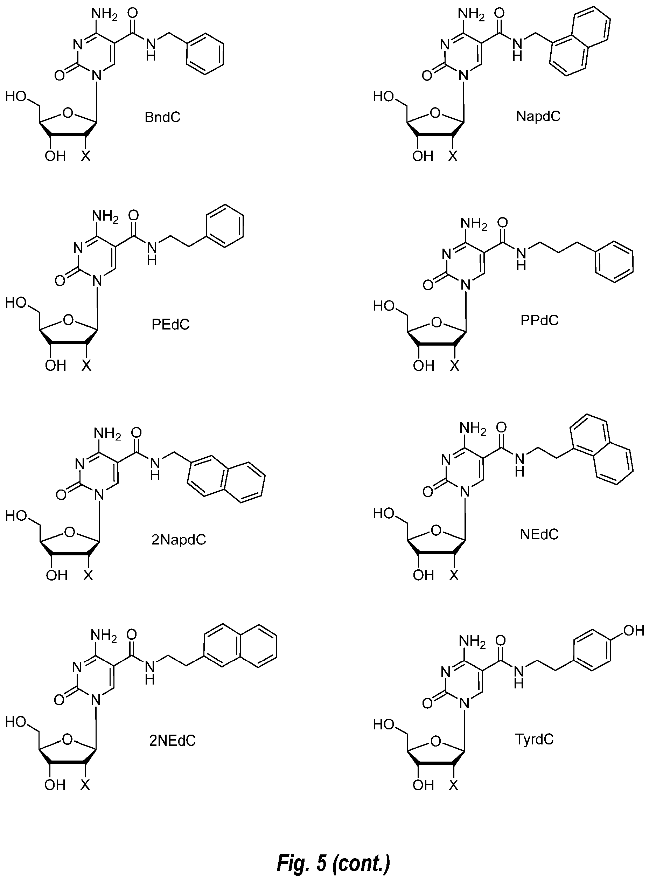

SELEX can also be used to identify aptamers that have desirable off-rate characteristics. See U.S. Publication No. 20090004667, entitled "Method for Generating Aptamers with Improved Off-Rates", which describes improved SELEX methods for generating aptamers that can bind to target molecules. Methods for producing aptamers and photoaptamers having slower rates of dissociation from their respective target molecules are described. The methods involve contacting the candidate mixture with the target molecule, allowing the formation of nucleic acid-target complexes to occur, and performing a slow off-rate enrichment process wherein nucleic acid-target complexes with fast dissociation rates will dissociate and not reform, while complexes with slow dissociation rates will remain intact. Additionally, the methods include the use of modified nucleotides in the production of candidate nucleic acid mixtures to generate aptamers with improved off-rate performance. Nonlimiting exemplary modified nucleotides include, for example, the modified pyrimidines shown in Figs. 3-5. In some embodiments, an aptamer comprises at least one nucleotide with a modification, such as a base modification. In some embodiments, an aptamer comprises at least one nucleotide with a hydrophobic modification, such as a hydrophobic base modification, allowing for hydrophobic contacts with a target protein. Such hydrophobic contacts, in some embodiments, contribute to greater affinity and/or slower off-rate binding by the aptamer. Nonlimiting exemplary nucleotides with hydrophobic modifications are shown in Figure 3. In some embodiments, an aptamer comprises at least 2, at least 3, at least 4, at least 5, at least 6, at least 7, at least 8, at least 9, or at least 10 nucleotides with hydrophobic modifications, where each hydrophobic modification may be the same or different from the others. In some embodiments, at least 1, at least 2, at least 3, at least 4, at least 5, at least 6, at least 7, at least 8, at least 9, or at least 10 hydrophobic modifications in an aptamer may be independently selected from the hydrophobic modifications shown in Figure 3.

In some embodiments, the aptamer is a slow off-rate aptamer. In some embodiments, a slow off-rate aptamer (including an aptamer comprising at least one nucleotide with a hydrophobic modification) has an off-rate (t½) of ≥ 30 minutes, ≥ 60 minutes, ≥ 90 minutes, ≥ 120 minutes, ≥ 150 minutes, ≥ 180 minutes, ≥ 210 minutes, or ≥ 240 minutes.

In some embodiments, an assay employs aptamers that include photoreactive functional groups that enable the aptamers to covalently bind or "photocrosslink" their target molecules. See, e.g., U.S. Patent No. 6,544,776 entitled "Nucleic Acid Ligand Diagnostic Biochip". These photoreactive aptamers are also referred to as photoaptamers. See, e.g., U.S. Patent No. 5,763,177, U.S. Patent No. 6,001,577, and U.S. Patent No. 6,291,184, each of which is entitled "Systematic Evolution of Nucleic Acid Ligands by Exponential Enrichment: Photoselection of Nucleic Acid Ligands and Solution SELEX"; see also, e.g., U.S. Patent No. 6,458,539, entitled "Photoselection of Nucleic Acid Ligands". After the microarray is contacted with the sample and the photoaptamers have had an opportunity to bind to their target molecules, the photoaptamers are photoactivated, and the solid support is washed to remove any non-specifically bound molecules. Harsh wash conditions may be used, since target molecules that are bound to the photoaptamers are generally not removed, due to the covalent bonds created by the photoactivated functional group(s) on the photoaptamers. In this manner, the assay enables the detection of a biomarker level corresponding to a biomarker in the test sample.

In some assay formats, the aptamers are immobilized on the solid support prior to being contacted with the sample. Under certain circumstances, however, immobilization of the aptamers prior to contact with the sample may not provide an optimal assay. For example, pre-immobilization of the aptamers may result in inefficient mixing of the aptamers with the target molecules on the surface of the solid support, perhaps leading to lengthy reaction times and, therefore, extended incubation periods to permit efficient binding of the aptamers to their target molecules. Further, when photoaptamers are employed in the assay and depending upon the material utilized as a solid support, the solid support may tend to scatter or absorb the light used to effect the formation of covalent bonds between the photoaptamers and their target molecules. Moreover, depending upon the method employed, detection of target molecules bound to their aptamers can be subject to imprecision, since the surface of the solid support may also be exposed to and affected by any labeling agents that are used. Finally, immobilization of the aptamers on the solid support generally involves an aptamer-preparation step (i.e., the immobilization) prior to exposure of the aptamers to the sample, and this preparation step may affect the activity or functionality of the aptamers.

Aptamer assays that permit an aptamer to capture its target in solution and then employ separation steps that are designed to remove specific components of the aptamer-target mixture prior to detection have also been described (see U.S. Publication No. 20090042206, entitled "Multiplexed Analyses of Test Samples"). The described aptamer assay methods enable the detection and quantification of a non-nucleic acid target (e.g., a protein target) in a test sample by detecting and quantifying a nucleic acid (i.e., an aptamer). The described methods create a nucleic acid surrogate (i.e, the aptamer) for detecting and quantifying a non-nucleic acid target, thus allowing the wide variety of nucleic acid technologies, including amplification, to be applied to a broader range of desired targets, including protein targets.

Aptamers can be constructed to facilitate the separation of the assay components from an aptamer biomarker complex (or photoaptamer biomarker covalent complex) and permit isolation of the aptamer for detection and/or quantification. In one embodiment, these constructs can include a cleavable or releasable element within the aptamer sequence. In other embodiments, additional functionality can be introduced into the aptamer, for example, a labeled or detectable component, a spacer component, or a specific binding tag or immobilization element. For example, the aptamer can include a tag connected to the aptamer via a cleavable moiety, a label, a spacer component separating the label, and the cleavable moiety. In one embodiment, a cleavable element is a photocleavable linker. The photocleavable linker can be attached to a biotin moiety and a spacer section, can include an NHS group for derivatization of amines, and can be used to introduce a biotin group to an aptamer, thereby allowing for the release of the aptamer later in an assay method.

Homogenous assays, done with all assay components in solution, do not require separation of sample and reagents prior to the detection of signal. These methods are rapid and easy to use. These methods generate signal based on a molecular capture or binding reagent that reacts with its specific target. In some embodiments, the molecular capture reagents comprise one or more aptamers and/or antibodies or the like and the specific target of each of the one or more aptamers and/or antibodies or the like may be a biomarker shown in Table 1.

In some embodiments, a method for signal generation takes advantage of anisotropy signal change due to the interaction of a fluorophore-labeled capture reagent with its specific biomarker target. When the labeled capture reacts with its target, the increased molecular weight causes the rotational motion of the fluorophore attached to the complex to become much slower changing the anisotropy value. By monitoring the anisotropy change, binding events may be used to quantitatively measure the biomarkers in solutions. Other methods include fluorescence polarization assays, molecular beacon methods, time resolved fluorescence quenching, chemiluminescence, fluorescence resonance energy transfer, and the like.

An exemplary solution-based aptamer assay that can be used to detect a biomarker level in a biological sample includes the following: (a) preparing a mixture by contacting the biological sample with an aptamer that includes a first tag and has a specific affinity for the biomarker, wherein an aptamer affinity complex is formed when the biomarker is present in the sample; (b) exposing the mixture to a first solid support including a first capture element, and allowing the first tag to associate with the first capture element; (c) removing any components of the mixture not associated with the first solid support; (d) attaching a second tag to the biomarker component of the aptamer affinity complex; I releasing the aptamer affinity complex from the first solid support; (f) exposing the released aptamer affinity complex to a second solid support that includes a second capture element and allowing the second tag to associate with the second capture element; (g) removing any non-complexed aptamer from the mixture by partitioning the non-complexed aptamer from the aptamer affinity complex; (h) eluting the aptamer from the solid support; and (i) detecting the biomarker by detecting the aptamer component of the aptamer affinity complex.

Any means known in the art can be used to detect a biomarker value by detecting the aptamer component of an aptamer affinity complex. A number of different detection methods can be used to detect the aptamer component of an affinity complex, such as, for example, hybridization assays, mass spectroscopy, or QPCR. In some embodiments, nucleic acid sequencing methods can be used to detect the aptamer component of an aptamer affinity complex and thereby detect a biomarker value. Briefly, a test sample can be subjected to any kind of nucleic acid sequencing method to identify and quantify the sequence or sequences of one or more aptamers present in the test sample. In some embodiments, the sequence includes the entire aptamer molecule or any portion of the molecule that may be used to uniquely identify the molecule. In other embodiments, the identifying sequencing is a specific sequence added to the aptamer; such sequences are often referred to as "tags," "barcodes," or "zipcodes." In some embodiments, the sequencing method includes enzymatic steps to amplify the aptamer sequence or to convert any kind of nucleic acid, including RNA and DNA that contain chemical modifications to any position, to any other kind of nucleic acid appropriate for sequencing.

In some embodiments, the sequencing method includes one or more cloning steps. In other embodiments the sequencing method includes a direct sequencing method without cloning.

In some embodiments, the sequencing method includes a directed approach with specific primers that target one or more aptamers in the test sample. In other embodiments, the sequencing method includes a shotgun approach that targets all aptamers in the test sample.

In some embodiments, the sequencing method includes enzymatic steps to amplify the molecule targeted for sequencing. In other embodiments, the sequencing method directly sequences single molecules. An exemplary nucleic acid sequencing-based method that can be used to detect a biomarker value corresponding to a biomarker in a biological sample includes the following: (a) converting a mixture of aptamers that contain chemically modified nucleotides to unmodified nucleic acids with an enzymatic step; (b) shotgun sequencing the resulting unmodified nucleic acids with a massively parallel sequencing platform such as, for example, the 454 Sequencing System (454 Life Sciences/Roche), the Illumina Sequencing System (Illumina), the ABI SOLiD Sequencing System (Applied Biosystems), the HeliScope Single Molecule Sequencer (Helicos Biosciences), or the Pacific Biosciences Real Time Single-Molecule Sequencing System (Pacific BioSciences) or the Polonator G Sequencing System (Dover Systems); and (c) identifying and quantifying the aptamers present in the mixture by specific sequence and sequence count.

A nonlimiting exemplary method of detecting biomarkers in a biological sample using aptamers is described in Example 1. *See also* Kraemer et al., 2011, PloS One 6(10): e26332.

### Determination of Biomarker Levels using Immunoassays

Immunoassay methods are based on the reaction of an antibody to its corresponding target or analyte and can detect the analyte in a sample depending on the specific assay format. To improve specificity and sensitivity of an assay method based on 22ffibo-reactivity, monoclonal antibodies and fragments thereof are often used because of their specific epitope recognition. Polyclonal antibodies have also been successfully used in various immunoassays because of their increased affinity for the target as compared to monoclonal antibodies. Immunoassays have been designed for use with a wide range of biological sample matrices. Immunoassay formats have been designed to provide qualitative, semi-quantitative, and quantitative results.

Quantitative results are generated through the use of a standard curve created with known concentrations of the specific analyte to be detected. The response or signal from an unknown sample is plotted onto the standard curve, and a quantity or level corresponding to the target in the unknown sample is established.

Numerous immunoassay formats have been designed. ELISA or EIA can be quantitative for the detection of an analyte. This method relies on attachment of a label to either the analyte or the antibody and the label component includes, either directly or indirectly, an enzyme. ELISA tests may be formatted for direct, indirect, competitive, or sandwich detection of the analyte. Other methods rely on labels such as, for example, radioisotopes (I¹²⁵) or fluorescence. Additional techniques include, for example, agglutination, nephelometry, turbidimetry, Western blot, immunoprecipitation, immunocytochemistry, immunohistochemistry, flow cytometry, Luminex assay, and others (see ImmunoAssay: A Practical Guide, edited by Brian Law, published by Taylor & Francis, Ltd., 2005 edition).

Exemplary assay formats include enzyme-linked immunosorbent assay (ELISA), radioimmunoassay, fluorescent, chemiluminescence, and fluorescence resonance energy transfer (FRET) or time resolved-FRET (TR-FRET) immunoassays. Examples of procedures for detecting biomarkers include biomarker immunoprecipitation followed by quantitative methods that allow size and peptide level discrimination, such as gel electrophoresis, capillary electrophoresis, planar electrochromatography, and the like.

Methods of detecting and/or for quantifying a detectable label or signal generating material depend on the nature of the label. The products of reactions catalyzed by appropriate enzymes (where the detectable label is an enzyme; see above) can be, without limitation, fluorescent, luminescent, or radioactive or they may absorb visible or ultraviolet light. Examples of detectors suitable for detecting such detectable labels include, without limitation, x-ray film, radioactivity counters, scintillation counters, spectrophotometers, colorimeters, fluorometers, luminometers, and densitometers.

Any of the methods for detection can be performed in any format that allows for any suitable preparation, processing, and analysis of the reactions. This can be, for example, in multi-well assay plates (e.g., 96 wells or 386 wells) or using any suitable array or microarray. Stock solutions for various agents can be made manually or robotically, and all subsequent pipetting, diluting, mixing, distribution, washing, incubating, sample readout, data collection and analysis can be done robotically using commercially available analysis software, robotics, and detection instrumentation capable of detecting a detectable label.

### Determination of Biomarker Levels using Gene Expression Profiling

This section is useful for understanding the invention, but the claimed invention involves measuring proteins. Measuring mRNA in a biological sample may, in some embodiments, be used as a surrogate for detection of the level of the corresponding protein in the biological sample. Thus, in some embodiments, a biomarker or biomarker panel described herein can be detected by detecting the appropriate RNA.

In some embodiments, mRNA expression levels are measured by reverse transcription quantitative polymerase chain reaction (RT-PCR followed with qPCR). RT-PCR is used to create a cDNA from the mRNA. The cDNA may be used in a qPCR assay to produce fluorescence as the DNA amplification process progresses. By comparison to a standard curve, qPCR can produce an absolute measurement such as number of copies of mRNA per cell. Northern blots, microarrays, Invader assays, and RT-PCR combined with capillary electrophoresis have all been used to measure expression levels of mRNA in a sample. See Gene Expression Profiling: Methods and Protocols, Richard A. Shimkets, editor, Humana Press, 2004.

### Detection of Biomarkers Using In Vivo Molecular Imaging Technologies

This section is useful for understanding the invention but the claimed invention involves measuring biomarkers in a plasma sample. In some embodiments not encompassed by the claims, a biomarker described herein may be used in molecular imaging tests. For example, an imaging agent can be coupled to a capture reagent, which can be used to detect the biomarker *in vivo.*

*In vivo* imaging technologies provide non-invasive methods for determining the state of a particular disease in the body of an individual. For example, entire portions of the body, or even the entire body, may be viewed as a three dimensional image, thereby providing valuable information concerning morphology and structures in the body. Such technologies may be combined with the detection of the biomarkers described herein to provide information concerning the biomarker *in vivo.*

The use of *in vivo* molecular imaging technologies is expanding due to various advances in technology. These advances include the development of new contrast agents or labels, such as radiolabels and/or fluorescent labels, which can provide strong signals within the body; and the development of powerful new imaging technology, which can detect and analyze these signals from outside the body, with sufficient sensitivity and accuracy to provide useful information. The contrast agent can be visualized in an appropriate imaging system, thereby providing an image of the portion or portions of the body in which the contrast agent is located. The contrast agent may be bound to or associated with a capture reagent, such as an aptamer or an antibody, for example, and/or with a peptide or protein, or an oligonucleotide (for example, for the detection of gene expression), or a complex containing any of these with one or more macromolecules and/or other particulate forms.

The contrast agent may also feature a radioactive atom that is useful in imaging. Suitable radioactive atoms include technetium-99m or iodine-123 for scintigraphic studies. Other readily detectable moieties include, for example, spin labels for magnetic resonance imaging (MRI) such as, for example, iodine-123 again, iodine-131, indium-111, fluorine-19, carbon-13, nitrogen-15, oxygen-17, gadolinium, manganese or iron. Such labels are well known in the art and could easily be selected by one of ordinary skill in the art.

Standard imaging techniques include but are not limited to magnetic resonance imaging, computed tomography scanning, positron emission tomography (PET), single photon emission computed tomography (SPECT), and the like. For diagnostic *in vivo* imaging, the type of detection instrument available is a major factor in selecting a given contrast agent, such as a given radionuclide and the particular biomarker that it is used to target (protein, mRNA, and the like). The radionuclide chosen typically has a type of decay that is detectable by a given type of instrument. Also, when selecting a radionuclide for *in vivo* diagnosis, its half-life should be long enough to enable detection at the time of maximum uptake by the target tissue but short enough that deleterious radiation of the host is minimized.

Exemplary imaging techniques include but are not limited to PET and SPECT, which are imaging techniques in which a radionuclide is synthetically or locally administered to an individual. The subsequent uptake of the radiotracer is measured over time and used to obtain information about the targeted tissue and the biomarker. Because of the high-energy (gamma-ray) emissions of the specific isotopes employed and the sensitivity and sophistication of the instruments used to detect them, the two-dimensional distribution of radioactivity may be inferred from outside of the body.

Commonly used positron-emitting nuclides in PET include, for example, carbon-11, nitrogen-13, oxygen-15, and fluorine-18. Isotopes that decay by electron capture and/or gamma-emission are used in SPECT and include, for example iodine-123 and technetium-99m. An exemplary method for labeling amino acids with technetium-99m is the reduction of pertechnetate ion in the presence of a chelating precursor to form the labile technetium-99m-precursor complex, which, in turn, reacts with the metal binding group of a bifunctionally modified chemotactic peptide to form a technetium-99m-chemotactic peptide conjugate.

Antibodies are frequently used for such *in vivo* imaging diagnostic methods. The preparation and use of antibodies for *in vivo* diagnosis is well known in the art. Similarly, aptamers may be used for such *in vivo* imaging diagnostic methods. For example, an aptamer that was used to identify a particular biomarker described herein may be appropriately labeled and injected into an individual to detect the biomarker *in vivo.* The label used will be selected in accordance with the imaging modality to be used, as previously described. Aptamer-directed imaging agents could have unique and advantageous characteristics relating to tissue penetration, tissue distribution, kinetics, elimination, potency, and selectivity as compared to other imaging agents.

Such techniques may also optionally be performed with labeled oligonucleotides, for example, for detection of gene expression through imaging with antisense oligonucleotides. These methods are used for in situ hybridization, for example, with fluorescent molecules or radionuclides as the label. Other methods for detection of gene expression include, for example, detection of the activity of a reporter gene.

Another general type of imaging technology is optical imaging, in which fluorescent signals within the subject are detected by an optical device that is external to the subject. These signals may be due to actual fluorescence and/or to bioluminescence. Improvements in the sensitivity of optical detection devices have increased the usefulness of optical imaging for in vivo diagnostic assays.

For a review of other techniques, see N. Blow, Nature Methods, 6, 465-469, 2009.

### Determination of Biomarker Levels using Mass Spectrometry Methods

A variety of configurations of mass spectrometers can be used to detect biomarker levels. Several types of mass spectrometers are available or can be produced with various configurations. In general, a mass spectrometer has the following major components: a sample inlet, an ion source, a mass analyzer, a detector, a vacuum system, and instrumentcontrol system, and a data system. Difference in the sample inlet, ion source, and mass analyzer generally define the type of instrument and its capabilities. For example, an inlet can be a capillary-column liquid chromatography source or can be a direct probe or stage such as used in matrix-assisted laser desorption. Common ion sources are, for example, electrospray, including nanospray and microspray or matrix-assisted laser desorption. Common mass analyzers include a quadrupole mass filter, ion trap mass analyzer and time-of-flight mass analyzer. Additional mass spectrometry methods are well known in the art (see Burlingame et al. Anal. Chem. 70:647 R-716R (1998); Kinter and Sherman, New York (2000)).

Protein biomarkers and biomarker levels can be detected and measured by any of the following: electrospray ionization mass spectrometry (ESI-MS), ESI-MS/MS, ESI-MS/(MS)n, matrix-assisted laser desorption ionization time-of-flight mass spectrometry (MALDI-TOF-MS), surface-enhanced laser desorption/ionization time-of-flight mass spectrometry (SELDI-TOF-MS), desorption/ionization on silicon (DIOS), secondary ion mass spectrometry (SIMS), quadrupole time-of-flight (Q-TOF), tandem time-of-flight (TOF/TOF) technology, called ultraflex III TOF/TOF, atmospheric pressure chemical ionization mass spectrometry (APCI-MS), APCI-MS/MS, APCI-(MS)N, atmospheric pressure photoionization mass spectrometry (APPI-MS), APPI-MS/MS, and APPI-(MS)N, quadrupole mass spectrometry, Fourier transform mass spectrometry (FTMS), quantitative mass spectrometry, and ion trap mass spectrometry.

Sample preparation and processing strategies are used to label and enrich samples before mass spectroscopic characterization of protein biomarkers and determination biomarker levels. Labeling methods include but are not limited to isobaric tag for relative and absolute quantitation (iTRAQ) and stable isotope labeling with amino acids in cell culture (SILAC). Capture reagents used to selectively enrich samples for candidate biomarker proteins prior to mass spectroscopic analysis include but are not limited to aptamers, antibodies, nucleic acid probes, chimeras, small molecules, an F(ab')₂ fragment, a single chain antibody fragment, an Fv fragment, a single chain Fv fragment, a nucleic acid, a lectin, a ligand-binding receptor, affibodies, nanobodies, ankyrins, domain antibodies, alternative antibody scaffolds (e.g. diabodies etc) imprinted polymers, avimers, peptidomimetics, peptoids, peptide nucleic acids, threose nucleic acid, a hormone receptor, a cytokine receptor, and synthetic receptors, and modifications and fragments of these.

### Determination of Biomarker Levels using a Proximity Ligation Assay

A proximity ligation assay can be used to determine biomarker values. Briefly, a test sample is contacted with a pair of affinity probes that may be a pair of antibodies or a pair of aptamers, with each member of the pair extended with an oligonucleotide. The targets for the pair of affinity probes may be two distinct determinates on one protein or one determinate on each of two different proteins, which may exist as homo- or hetero-multimeric complexes. When probes bind to the target determinates, the free ends of the oligonucleotide extensions are brought into sufficiently close proximity to hybridize together. The hybridization of the oligonucleotide extensions is facilitated by a common connector oligonucleotide which serves to bridge together the oligonucleotide extensions when they are positioned in sufficient proximity. Once the oligonucleotide extensions of the probes are hybridized, the ends of the extensions are joined together by enzymatic DNA ligation.

Each oligonucleotide extension comprises a primer site for PCR amplification. Once the oligonucleotide extensions are ligated together, the oligonucleotides form a continuous DNA sequence which, through PCR amplification, reveals information regarding the identity and amount of the target protein, as well as, information regarding protein-protein interactions where the target determinates are on two different proteins. Proximity ligation can provide a highly sensitive and specific assay for real-time protein concentration and interaction information through use of real-time PCR. Probes that do not bind the determinates of interest do not have the corresponding oligonucleotide extensions brought into proximity and no ligation or PCR amplification can proceed, resulting in no signal being produced.

The foregoing assays enable the detection of biomarker values that are useful in methods for assessing fasting time, wherein the methods comprise detecting, in a biological sample from an individual, at least 2, at least 3, at least 4, at least 5, at least 6, or all 7 biomarkers selected from the biomarkers in Table 1. A classification, as described below, using the biomarker levels indicates whether the sample has acceptable quality for use in subsequent analysis. In accordance with any of the methods described herein, biomarker levels can be detected and classified individually or they can be detected and classified collectively, as for example in a multiplex assay format.

### Classification of Biomarkers and Calculation of Fasting Times

In some embodiments, a biomarker "signature" for a given sample quality test contains a set of biomarkers, each biomarker having characteristic levels in samples of acceptable quality or in sample of poor quality. Characteristic levels, in some embodiments, may refer to the mean or average of the biomarker levels for the samples in a particular group. In some embodiments, a method described herein can be used to assign a sample into one of two groups, either passing quality assessment of failing quality assessment.

The assignment of a sample into one of two or more groups is known as classification, and the procedure used to accomplish this assignment is known as a classifier or a classification method. Classification methods may also be referred to as scoring methods. There are many classification methods that can be used to construct a classifier from a set of biomarker levels. In some instances, classification methods are performed using supervised learning techniques in which a data set is collected using samples from two (or more, for multiple classification states) distinct groups one wishes to distinguish. Since the class (group or population) to which each sample belongs is known in advance for each sample, the classification method can be trained to give the desired classification response. It is also possible to use unsupervised learning techniques to produce a quality classifier.

Common approaches for developing classifiers include decision trees; bagging + boosting + forests; rule inference based learning; Parzen Windows; linear models; logistic; neural network methods; unsupervised clustering; K-means; hierarchical ascending/ descending; semi-supervised learning; prototype methods; nearest neighbor; kernel density estimation; support vector machines; hidden Markov models; Boltzmann Learning; and classifiers may be combined either simply or in ways which minimize particular objective functions. For a review, see, e.g., Pattern Classification, R.O. Duda, et al., editors, John Wiley & Sons, 2nd edition, 2001; see also, The Elements of Statistical Learning - Data Mining, Inference, and Prediction, T. Hastie, et al., editors, Springer Science+Business Media, LLC, 2nd edition, 2009.

To produce a classifier using supervised learning techniques, a set of samples called training data are obtained. In the context of quality tests, training data includes samples from the distinct groups (classes) to which unknown samples will later be assigned. For example, samples that underwent processing with differing, set times between processing steps can constitute training data to develop a classifier that can classify unknown samples as either passing or failing quality assessment based on the elapsed fasting time between a meal and sample collection. The development of the classifier from the training data is known as training the classifier. Specific details on classifier training depend on the nature of the supervised learning technique. Training a naive Bayesian classifier is an example of such a supervised learning technique (see, e.g., Pattern Classification, R.O. Duda, et al., editors, John Wiley & Sons, 2nd edition, 2001; see also, The Elements of Statistical Learning - Data Mining, Inference, and Prediction, T. Hastie, et al., editors, Springer Science+Business Media, LLC, 2nd edition, 2009). Training of a naive Bayesian classifier is described, e.g., in U.S. Publication Nos: 2012/0101002 and 2012/0077695.

Since typically there are many more potential biomarker levels than samples in a training set, care must be used to avoid over-fitting. Over-fitting occurs when a statistical model describes random error or noise instead of the underlying relationship. Over-fitting can be avoided in a variety of way, including, for example, by limiting the number of biomarkers used in developing the classifier, by assuming that the biomarker responses are independent of one another, by limiting the complexity of the underlying statistical model employed, and by ensuring that the underlying statistical model conforms to the data.

An illustrative example of the development of a test using a set of biomarkers includes the application of a naive Bayes classifier, a simple probabilistic classifier based on Bayes theorem with strict independent treatment of the biomarkers. Each biomarker is described by a class-dependent probability density function (pdf) for the measured RFU values or log RFU (relative fluorescence units) values in each class. The joint pdfs for the set of biomarkers in one class is assumed to be the product of the individual class-dependent pdfs for each biomarker. Training a naive Bayes classifier in this context amounts to assigning parameters ("parameterization") to characterize the class dependent pdfs. Any underlying model for the class-dependent pdfs may be used, but the model should generally conform to the data observed in the training set.

The performance of the naive Bayes classifier is dependent upon the number and quality of the biomarkers used to construct and train the classifier. A single biomarker will perform in accordance with its KS-distance (Kolmogorov-Smirnov). The addition of subsequent biomarkers with good KS distances (>0.3, for example) will, in general, improve the classification performance if the subsequently added biomarkers are independent of the first biomarker. Using the sensitivity plus specificity as a classifier score, many high scoring classifiers can be generated with a variation of a greedy algorithm. (A greedy algorithm is any algorithm that follows the problem solving metaheuristic of making the locally optimal choice at each stage with the hope of finding the global optimum.)

Another way to depict classifier performance is through a receiver operating characteristic (ROC), or simply ROC curve or ROC plot. The ROC is a graphical plot of the sensitivity, or true positive rate, vs. false positive rate (1 - specificity or 1 - true negative rate), for a binary classifier system as its discrimination threshold is varied. The ROC can also be represented equivalently by plotting the fraction of true positives out of the positives (TPR = true positive rate) vs. the fraction of false positives out of the negatives (FPR = false positive rate). Also known as a Relative Operating Characteristic curve, because it is a comparison of two operating characteristics (TPR & FPR) as the criterion changes. The area under the ROC curve (AUC) is commonly used as a summary measure of diagnostic accuracy. It can take values from 0.0 to 1.0. The AUC has an important statistical property: the AUC of a classifier is equivalent to the probability that the classifier will rank a randomly chosen positive instance higher than a randomly chosen negative instance (Fawcett T, 2006. An introduction to ROC analysis. Pattern Recognition Letters .27: 861-874). This is equivalent to the Wilcoxon test of ranks (Hanley, J.A., McNeil, B.J., 1982. The meaning and use of the area under a receiver operating characteristic (ROC) curve. Radiology 143, 29-36.). Another way of describing performance of a diagnostic test in relation to a known reference standard is the net reclassification index: the ability of the new test to correctly upgrade or downgrade risk when compared with the reference standard test. See, e.g., Pencina et al., 2011, Stat. Med. 30: 11-21. While the AUC under the ROC curve is optimal for assessing performance of a 2-class classifier, stratified and personalized medicine relies upon the inference that the population contains more classes than 2. For such comparisons the hazard ratio of the upper vs. lower quartiles (or other stratifications such as deciles) can be used more appropriately.

### Kits

Kits are not claimed as such but may be useful for performing the invention. Any combination of the biomarkers described herein can be detected using a suitable kit, such as for use in performing the methods disclosed herein. Furthermore, any kit can contain one or more detectable labels as described herein, such as a fluorescent moiety, etc.

In some embodiments not encompassed by the claims, a kit includes (a) one or more capture reagents (such as, for example, at least one aptamer or antibody) for detecting one or more biomarkers in a biological sample, wherein the biomarkers include at least 2, at least 3, at least 4, at least 5, at least 6, or all 7 biomarkers selected from the biomarkers in Table 1; and optionally (b) one or more software or computer program products for classifying the sample was obtained as either passing or failing quality assessment or for determining the approximate time or times of fasting, as further described herein. Alternatively, rather than one or more computer program products, one or more instructions for manually performing the above steps by a human can be provided.

In some embodiments, a kit comprises a solid support, at least one capture reagent, and a signal generating material. The kit can also include instructions for using the devices and reagents, handling the sample, and analyzing the data. Further the kit may be used with a computer system or software to analyze and report the result of the analysis of the biological sample.

The kit may further comprise reagents for diagnostic analysis of samples, particularly samples that pass quality assessment.

The kits can also contain one or more reagents (e.g., solubilization buffers, detergents, washes, or buffers) for processing a biological sample. Any of the kits described herein can also include, e.g., buffers, blocking agents, mass spectrometry matrix materials, antibody capture agents, positive control samples, negative control samples, software and information such as protocols, guidance and reference data.

In some embodiments, the kits comprise PCR primers for one or more aptamers specific to biomarkers described herein. In some embodiments, a kit may further include instructions for use and correlation of the biomarkers with estimation of fasting times and/or sample quality. In some embodiments, a kit may also include a DNA array containing the complement of one or more of the aptamers specific for the biomarkers described herein, reagents, and/or enzymes for amplifying or isolating sample DNA. In some embodiments, kits may include reagents for real-time PCR, for example, TaqMan probes and/or primers, and enzymes.

For example, a kit can comprise (a) reagents comprising at least one capture reagent for determining the level of one or more biomarkers in a test sample, and optionally (b) one or more algorithms or computer programs for performing the steps of comparing the amount of each biomarker quantified in the test sample to one or more predetermined cutoffs. In some embodiments, an algorithm or computer program assigns a score for each biomarker quantified based on the comparison and, in some embodiments, combines the assigned scores for each biomarker quantified to obtain a total score. Further, in some embodiments, an algorithm or computer program compares the total score with a predetermined score, and uses the comparison to determine whether a sample passes or fails quality assessment. Alternatively, rather than one or more algorithms or computer programs, one or more instructions for manually performing the above steps by a human can be provided.

### Biomarker Panels

In some embodiments, the level of each protein listed in Table 1 is detected. In some embodiments, the sample processing comprises or consists of sample centrifugation, removal of the sample supernatant by decanting or aspirating and freezing. In some such embodiments, sample processing was performed immediately after sample collection from the subject.

**Table 1: Panel for fed-fasted plasma samples**

| **UniProt** | **Protein** | **Gene** | **Name** |
|---|---|---|---|
| P01275 | Glucagon | GCG | Glucagon |
| P09681 | GIP | GIP | Gastric inhibitory polypeptide |
| P07148 | FABPL | FABP1 | Fatty acid-binding protein, liver |
| Q14512 | FGFP1 | FGFBP 1 | Fibroblast growth factor-binding protein 1 |
| P01308 | Insulin | INS | Insulin |
| P01298 | PH | PPY | Pancreatic hormone |
| P49755 | TMEDA | TMED 10 | Transmembrane emp24 domain-containing protein 10 |

### Computer Methods and Software

Computer systems and software are not claimed as such but maybe useful for performing the invention. A method for assessing sample quality, such as the length of fasting time or approximate length of fasting time in hours that elapsed from a meal until sample collection can comprise the following: 1) obtain a biological sample, such as a sample that has already undergone sample processing; 2) perform an analytical method to detect and measure a biomarker or set of biomarkers in a panel in the biological sample; 3) optionally perform any data normalization or standardization; 4) determine each biomarker level; and 5) report the results. In some embodiments, the results are calibrated to the sample type. In some embodiments, the biomarker levels are combined in some way and a single value for the combined biomarker levels is reported. In this approach, in some embodiments, the score may be a single number or identity determined from the integration of all the biomarkers that is compared to a pre-set threshold value that is an indication of satisfactory (passing) or unsatisfactory (failing) quality. Or the predictive score may be a series of bars that each represent a biomarker value and the pattern of the responses may be compared to a pre-set pattern for determination of the satisfactory (passing) or unsatisfactory (failing) quality.

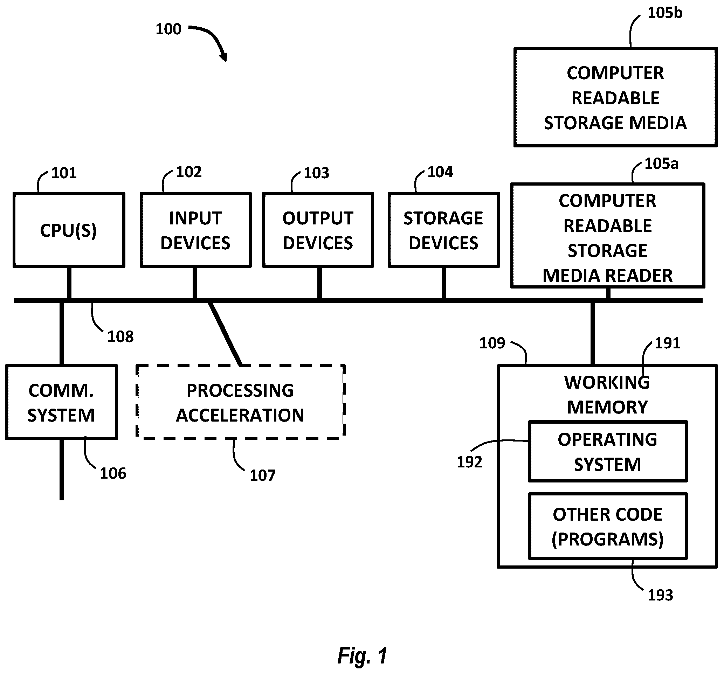

At least some embodiments of the methods described herein can be implemented with the use of a computer. An example of a computer system 100 is shown in Figure 1. With reference to Figure 1, system 100 is shown comprised of hardware elements that are electrically coupled via bus 108, including a processor 101, input device 102, output device 103, storage device 104, computer-readable storage media reader 105a, communications system 106, processing acceleration (e.g., DSP or special-purpose processors) 107 and memory 109. Computer-readable storage media reader 105a is further coupled to computer-readable storage media 105b, the combination comprehensively representing remote, local, fixed and/or removable storage devices plus storage media, memory, etc. for temporarily and/or more permanently containing computer-readable information, which can include storage device 104, memory 109 and/or any other such accessible system 100 resource. System 100 also comprises software elements (shown as being currently located within working memory 191) including an operating system 192 and other code 193, such as programs, data and the like.

With respect to Figure 1, system 100 has extensive flexibility and configurability. Thus, for example, a single architecture might be utilized to implement one or more servers that can be further configured in accordance with currently desirable protocols, protocol variations, extensions, etc. However, it will be apparent to those skilled in the art that embodiments may well be utilized in accordance with more specific application requirements. For example, one or more system elements might be implemented as subelements within a system 100 component (e.g., within communications system 106). Customized hardware might also be utilized and/or particular elements might be implemented in hardware, software or both. Further, while connection to other computing devices such as network input/output devices (not shown) may be employed, it is to be understood that wired, wireless, modem, and/or other connection or connections to other computing devices might also be utilized.

In one aspect, the system can comprise a database containing features of biomarkers characteristic of sample quality. The biomarker data (or biomarker information) can be utilized as an input to the computer for use as part of a computer implemented method. The biomarker data can include the data as described herein.

In one aspect, the system further comprises one or more devices for providing input data to the one or more processors.

The system further comprises a memory for storing a data set of ranked data elements.

In another aspect, the device for providing input data comprises a detector for detecting the characteristic of the data element, e.g., such as a mass spectrometer or gene chip reader.

The system additionally may comprise a database management system. User requests or queries can be formatted in an appropriate language understood by the database management system that processes the query to extract the relevant information from the database of training sets.

The system may be connectable to a network to which a network server and one or more clients are connected. The network may be a local area network (LAN) or a wide area network (WAN), as is known in the art. Preferably, the server includes the hardware necessary for running computer program products (e.g., software) to access database data for processing user requests.

The system may include an operating system (e.g., UNIX or Linux) for executing instructions from a database management system. In one aspect, the operating system can operate on a global communications network, such as the internet, and utilize a global communications network server to connect to such a network.

The system may include one or more devices that comprise a graphical display interface comprising interface elements such as buttons, pull down menus, scroll bars, fields for entering text, and the like as are routinely found in graphical user interfaces known in the art. Requests entered on a user interface can be transmitted to an application program in the system for formatting to search for relevant information in one or more of the system databases. Requests or queries entered by a user may be constructed in any suitable database language.

The graphical user interface may be generated by a graphical user interface code as part of the operating system and can be used to input data and/or to display inputted data. The result of processed data can be displayed in the interface, printed on a printer in communication with the system, saved in a memory device, and/or transmitted over the network or can be provided in the form of the computer readable medium.

The system can be in communication with an input device for providing data regarding data elements to the system (e.g., expression values). In one aspect, the input device can include a gene expression profiling system including, e.g., a mass spectrometer, gene chip or array reader, and the like.

The methods and apparatus for analyzing sample quality biomarker information according to various embodiments may be implemented in any suitable manner, for example, using a computer program operating on a computer system. A conventional computer system comprising a processor and a random access memory, such as a remotelyaccessible application server, network server, personal computer or workstation may be used. Additional computer system components may include memory devices or information storage systems, such as a mass storage system and a user interface, for example a conventional monitor, keyboard and tracking device. The computer system may be a stand-alone system or part of a network of computers including a server and one or more databases.

The sample quality biomarker analysis system can provide functions and operations to complete data analysis, such as data gathering, processing, analysis, reporting and/or sample quality identification. For example, in one embodiment, the computer system can execute the computer program that may receive, store, search, analyze, and report information relating to the sample quality assessment biomarkers. The computer program may comprise multiple modules performing various functions or operations, such as a processing module for processing raw data and generating supplemental data and an analysis module for analyzing raw data and supplemental data to generate a sample quality status and/or estimated fasting time calculation. Calculation of fasting time may optionally comprise generating or collecting additional information.

Some embodiments described herein can be implemented so as to include a computer program product. A computer program product may include a computer readable medium having computer readable program code embodied in the medium for causing an application program to execute on a computer with a database.

As used herein, a "computer program product" refers to an organized set of instructions in the form of natural or programming language statements that are contained on a physical media of any nature (e.g., written, electronic, magnetic, optical or otherwise) and that may be used with a computer or other automated data processing system. Such programming language statements, when executed by a computer or data processing system, cause the computer or data processing system to act in accordance with the particular content of the statements. Computer program products include without limitation: programs in source and object code and/or test or data libraries embedded in a computer readable medium. Furthermore, the computer program product that enables a computer system or data processing equipment device to act in pre-selected ways may be provided in a number of forms, including, but not limited to, original source code, assembly code, object code, machine language, encrypted or compressed versions of the foregoing and any and all equivalents.

In one aspect not encompassed by the claims, a computer program product is provided for evaluation of sample quality. The computer program product includes a computer readable medium embodying program code executable by a processor of a computing device or system, the program code comprising: code that retrieves data attributed to a biological sample from an individual, wherein the data comprises biomarker levels that each correspond to one of the biomarkers in Table 1; and code that executes a classification method that indicates a sample quality status as a function of the biomarker levels.

In still another aspect not encompassed by the claims, a computer program product is provided for determining a fasting time or times. The computer program product includes a computer readable medium embodying program code executable by a processor of a computing device or system, the program code comprising: code that retrieves data attributed to a biological sample from an individual, wherein the data comprises a biomarker value corresponding to at least one biomarker in the biological sample selected from the biomarkers provided in Table 1; and code that executes a classification method that indicates a sample quality status as a function of the biomarker levels.

While various embodiments have been described as methods or apparatuses, it should be understood that embodiments can be implemented through code coupled with a computer, e.g., code resident on a computer or accessible by the computer. For example, software and databases could be utilized to implement many of the methods discussed above. Thus, in addition to embodiments accomplished by hardware, it is also noted that these embodiments can be accomplished through the use of an article of manufacture comprised of a computer usable medium having a computer readable program code embodied therein, which causes the enablement of the functions disclosed in this description. Therefore, it is desired that embodiments also be considered protected by this patent in their program code means as well. Furthermore, the embodiments may be embodied as code stored in a computer-readable memory of virtually any kind including, without limitation, RAM, ROM, magnetic media, optical media, or magneto-optical media. Even more generally, the embodiments could be implemented in software, or in hardware, or any combination thereof including, but not limited to, software running on a general purpose processor, microcode, programmable logic arrays (PLAs), or application-specific integrated circuits (ASICs).

It is also envisioned that embodiments could be accomplished as computer signals embodied in a carrier wave, as well as signals (e.g., electrical and optical) propagated through a transmission medium. Thus, the various types of information discussed above could be formatted in a structure, such as a data structure, and transmitted as an electrical signal through a transmission medium or stored on a computer readable medium.

It is also noted that many of the structures, materials, and acts recited herein can be recited as means for performing a function or step for performing a function. Therefore, it should be understood that such language is entitled to cover all such structures, materials, or acts disclosed within this specification and their equivalents.

The utilization of the biomarkers disclosed herein, and the various methods for determining biomarker values are described in detail above with respect to evaluation of sample quality and suitability for further analysis, such as diagnostic analysis. In some embodiments, the biomarkers, methods, and kits described herein are used to assess the absolute sample quality of one or more samples or the relative consistency of sample quality across a plurality of samples. In some such embodiments, the methods comprise identifying samples that pass or fail a quality assessment. In some embodiments, samples that pass quality assessment are analyzed and samples that fail quality assessment are discarded. In some embodiments, information obtained using the biomarkers, methods, and kits herein can be used to determine whether a sample collection and processing method or facility is suitable.

### EXAMPLES

The following examples are provided for illustrative purposes only and are not intended to limit the scope of the application as defined by the appended claims. Routine molecular biology techniques described in the following examples can be carried out as described in standard laboratory manuals, such as Sambrook et al., Molecular Cloning: A Laboratory Manual, 3rd. ed., Cold Spring Harbor Laboratory Press, Cold Spring Harbor, N.Y., (2001).

### Example 1: Exemplary Biomarker Detection Using Aptamers

An exemplary method of detecting one or more biomarker proteins in a sample is described, e.g., in Kraemer et al., PloS One 6(10): e26332, and is described below. Three different methods of quantification: microarray-based hybridization, a Luminex beadbased method, and qPCR, are described.

### Reagents

HEPES, NaCl, KCl, EDTA, EGTA, MgCl₂ and Tween-20 may be purchased, e.g., from Fisher Biosciences. Dextran sulfate sodium salt (DxSO4), nominally 8000 molecular weight, may be purchased, e.g., from AIC and is dialyzed against deionized water for at least 20 hours with one exchange. KOD EX DNA polymerase may be purchased, e.g., from VWR. Tetramethylammonium chloride and CAPSO may be purchased, e.g., from Sigma-Aldrich and streptavidin-phycoerythrin (SAPE) may be purchased, e.g., from Moss Inc. 4-(2-Aminoethyl)-benzenesulfonylfluoride hydrochloride (AEBSF) may be purchased, e.g., from Gold Biotechnology. Streptavidin-coated 96-well plates may be purchased, e.g., from Thermo Scientific (Pierce Streptavidin Coated Plates HBC, clear, 96-well, product number 15500 or 15501). NHS-PEO4-biotin may be purchased, e.g., from Thermo Scientific (EZ-Link NHS-PEO4-Biotin, product number 21329), dissolved in anhydrous DMSO, and may be stored frozen in single-use aliquots. IL-8, MIP-4, Lipocalin-2, RANTES, MMP-7, and MMP-9 may be purchased, e.g., from R&D Systems. Resistin and MCP-1 may be purchased, e.g., from PeproTech, and tPA may be purchased, e.g., from VWR.

### Nucleic acids

Conventional (including amine- and biotin-substituted) oligodeoxynucleotides may be purchased, e.g., from Integrated DNA Technologies (IDT). Z-Block is a single-stranded oligodeoxynucleotide of sequence 5'- (AC-BnBn)7-AC-3', where Bn indicates a benzyl-substituted deoxyuridine residue. Z-block may be synthesized using conventional phosphoramidite chemistry. Aptamer capture reagents may also be synthesized by conventional phosphoramidite chemistry, and may be purified, for example, on a 21.5×75 mm PRP-3 column, operating at 80°C on a Waters Autopurification 2767 system (or Waters 600 series semi-automated system), using, for example, a timberline TL-600 or TL-150 heater and a gradient of triethylammonium bicarbonate (TEAB) / I to elute product. Detection is performed at 260 nm and fractions are collected across the main peak prior to pooling best fractions.

### Buffers

Buffer SB18 is composed of 40 mM HEPES, 101 mM NaCl, 5 mM KCl, 5 mM MgCl2, and 0.05% (v/v) Tween 20 adjusted to pH 7.5 with NaOH. Buffer SB17 is SB18 supplemented with 1 mM trisodium EDTA. Buffer PB1 is composed of 10 mM HEPES, 101 mM NaCl, 5 mM KCl, 5 mM MgCl2, 1 mM trisodium EDTA and 0.05% (v/v) Tween-20 adjusted to pH 7.5 with NaOH. CAPSO elution buffer consists of 100 mM CAPSO pH 10.0 and 1 M NaCl. Neutralization buffer contains of 500 mM HEPES, 500 mM HCl, and 0.05% (v/v) Tween-20. Agilent Hybridization Buffer is a proprietary formulation that is supplied as part of a kit (Oligo aCGH/ChIP-on-chip Hybridization Kit). Agilent Wash Buffer 1 is a proprietary formulation (Oligo aCGH/ChIP-on-chip Wash Buffer 1, Agilent). Agilent Wash Buffer 2 is a proprietary formulation (Oligo aCGH/ChIP-on-chip Wash Buffer 2, Agilent). TMAC hybridization solution consists of 4.5 M tetramethylammonium chloride, 6 mM trisodium EDTA, 75 mM Tris-HCl (pH 8.0), and 0.15% (v/v) Sarkosyl. KOD buffer (10-fold concentrated) consists of 1200 mM Tris-HCl, 15 mM MgSO4, 100 mM KCl, 60 mM (NH4)2SO4, 1% v/v Triton-X 100 and 1 mg/mL BSA.

### Sample preparation

Serum (stored at -80°C in 100 µL aliquots) is thawed in a 25°C water bath for 10 minutes, then stored on ice prior to sample dilution. Samples are mixed by gentle vortexing for 8 seconds. A 6% serum sample solution is prepared by dilution into 0.94× SB17 supplemented with 0.6 mM MgCl₂, 1 mM trisodium EGTA, 0.8 mM AEBSF, and 2 µM Z-Block. A portion of the 6% serum stock solution is diluted 10-fold in SB 17 to create a 0.6% serum stock. 6% and 0.6% stocks are used, in some embodiments, to detect high- and low-abundance analytes, respectively.

### Capture reagent (aptamer) and streptavidin plate preparation

Aptamers are grouped into 2 mixes according to the relative abundance of their cognate analytes (or biomarkers). Stock concentrations are 4 nM for each aptamer, and the final concentration of each aptamer is 0.5 nM. Aptamer stock mixes are diluted 4-fold in SB17 buffer, heated to 95 °C for 5 min and cooled to 37 °C over a 15-minute period prior to use. This denaturation-renaturation cycle is intended to normalize aptamer conformer distributions and thus ensure reproducible aptamer activity in spite of variable histories. Streptavidin plates are washed twice with 150 µL buffer PB1 prior to use.

### Incubation and plate capture

Heat-cooled 2× Aptamer mixes (55 µL) are combined with an equal volume of 6% or 0.6% serum dilutions, producing mixes containing 3% and 0.3% serum. The plates are sealed with a Silicone Sealing Mat (Axymat Silicone sealing mat, VWR) and incubated for 1.5 h at 37 °C. Mixes are then transferred to the wells of a washed 96-well streptavidin plate and further incubated on an Eppendorf Thermomixer set at 37 °C, with shaking at 800 rpm, for two hours.

### Manual Assay

Unless otherwise specified, liquid is removed by dumping, followed by two taps onto layered paper towels. Wash volumes are 150 µL and all shaking incubations are done on an Eppendorf Thermomixer set at 25°C, 800 rpm. Mixes are removed by pipetting, and plates are washed twice for 1 minute with buffer PB1 supplemented with 1 mM dextran sulfate and 500 µM biotin, then 4 times for 15 seconds with buffer PB1. A freshly made solution of 1 mM NHS-PEO4-biotin in buffer PB1 (150 µL/well) is added, and plates are incubated for 5 minutes with shaking. The NHS-biotin solution is removed, and plates washed 3 times with buffer PB1 supplemented with 20 mM glycine, and 3 times with buffer PB1. Eighty-five µL of buffer PB1 supplemented with 1 mM DxSO₄ is then added to each well, and plates are irradiated under a BlackRay UV lamp (nominal wavelength 365 nm) at a distance of 5 cm for 20 minutes with shaking. Samples are transferred to a fresh, washed streptavidin-coated plate, or an unused well of the existing washed streptavidin plate, combining high and low sample dilution mixtures into a single well. Samples are incubated at room temperature with shaking for 10 minutes. Unabsorbed material is removed and the plates washed 8 times for 15 seconds each with buffer PB1 supplemented with 30% glycerol. Plates are then washed once with buffer PB1. Aptamers are eluted for 5 minutes at room temperature with 100 µL CAPSO elution buffer. 90 µL of the eluate is transferred to a 96-well HybAid plate and 10 µL neutralization buffer is added.

### Semi-Automated Assay

Streptavidin plates bearing adsorbed equilibration mixes are placed on the deck of a BioTek EL406 plate washer, which is programmed to perform the following steps: unadsorbed material is removed by aspiration, and wells are washed 4 times with 300 µL of buffer PB1 supplemented with 1 mM dextran sulfate and 500 µM biotin. Wells are then washed 3 times with 300 µL buffer PB1. One hundred fifty µL of a freshly prepared (from a 100 mM stock in DMSO) solution of 1 mM NHS-PEO4-biotin in buffer PB1 is added. Plates are incubated for 5 minutes with shaking. Liquid is aspirated, and wells are washed 8 times with 300 µL buffer PB1 supplemented with 10 mM glycine. One hundred µL of buffer PB1 supplemented with 1 mM dextran sulfate are added. After these automated steps, plates are removed from the plate washer and placed on a thermoshaker mounted under a UV light source (BlackRay, nominal wavelength 365 nm) at a distance of 5 cm for 20 minutes. The thermoshaker is set at 800 rpm and 25 °C. After 20 minutes irradiation, samples are manually transferred to a fresh, washed streptavidin plate (or to an unused well of the existing washed plate). High-abundance (3% serum+3% aptamer mix) and low-abundance reaction mixes (0.3% serum+0.3% aptamer mix) are combined into a single well at this point. This "Catch-2" plate is placed on the deck of BioTek EL406 plate washer, which is programmed to perform the following steps: the plate is incubated for 10 minutes with shaking. Liquid is aspirated, and wells are washed 21 times with 300 µL buffer PB1 supplemented with 30% glycerol. Wells are washed 5 times with 300 µL buffer PB1, and the final wash is aspirated. One hundred µL CAPSO elution buffer are added, and aptamers are eluted for 5 minutes with shaking. Following these automated steps, the plate is then removed from the deck of the plate washer, and 90 µL aliquots of the samples are transferred manually to the wells of a HybAid 96-well plate that contains 10 µL neutralization buffer.

### Hybridization to custom Agilent 8×15k microarrays

24 µL of the neutralized eluate is transferred to a new 96-well plate and 6 µL of 10× Agilent Block (Oligo aCGH/ChIP-on-chip Hybridization Kit, Large Volume, Agilent 5188-5380), containing a set of hybridization controls composed of 10 Cy3 aptamers is added to each well. Thirty µL 2× Agilent Hybridization buffer is added to each sample and mixed. Forty µL of the resulting hybridization solution is manually pipetted into each "well" of the hybridization gasket slide (Hybridization Gasket Slide, 8-microarray per slide format, Agilent). Custom Agilent microarray slides, bearing 10 probes per array complementary to 40 nucleotide random region of each aptamer with a 20× dT linker, are placed onto the gasket slides according to the manufacturers' protocol. The assembly (Hybridization Chamber Kit - SureHyb-enabled, Agilent) is clamped and incubated for 19 hours at 60 °C while rotating at 20 rpm.

### Post Hybridization Washing

Approximately 400 mL Agilent Wash Buffer 1 is placed into each of two separate glass staining dishes. Slides (no more than two at a time) are disassembled and separated while submerged in Wash Buffer 1, then transferred to a slide rack in a second staining dish also containing Wash Buffer 1. Slides are incubated for an additional 5 minutes in Wash Buffer 1 with stirring. Slides are transferred to Wash Buffer 2 pre-equilibrated to 37 °C and incubated for 5 minutes with stirring. Slides are transferred to a fourth staining dish containing acetonitrile, and incubated for 5 minutes with stirring.

### Microarray Imaging

Microarray slides are imaged with an Agilent G2565CA Microarray Scanner System, using the Cy3-channel at 5 µm resolution at 100% PMT setting, and the XRD option enabled at 0.05. The resulting TIFF images are processed using Agilent feature extraction software version 10.5.1.1 with the GE1_105_Dec08 protocol.

### Luminex probe design

Probes immobilized to beads have 40 deoxynucleotides complementary to the 3' end of the 40 nucleotide random region of the target aptamer. The aptamer complementary region is coupled to Luminex Microspheres through a hexaethyleneglycol (HEG) linker bearing a 5' amino terminus. Biotinylated detection deoxyoligonucleotides comprise 17-21 deoxynucleotides complementary to the 5' primer region of target aptamers. Biotin moieties are appended to the 3' ends of detection oligos.

### Coupling of probes to Luminex Microspheres

Probes are coupled to Luminex Microplex Microspheres essentially per the manufacturer's instructions, but with the following modifications: amino-terminal oligonucleotide amounts are 0.08 nMol per 2.5×10⁶ microspheres, and the second EDC addition is 5 µL at 10 mg/mL. Coupling reactions are performed in an Eppendorf ThermoShaker set at 25 °C and 600 rpm.

### Microsphere hybridization

Microsphere stock solutions (about 40000 microspheres/µL) are vortexed and sonicated in a Health Sonics ultrasonic cleaner (Model: T1.9C) for 60 seconds to suspend the microspheres. Suspended microspheres are diluted to 2000 microspheres per reaction in 1.5× TMAC hybridization solutions and mixed by vortexing and sonication. Thirty-three µL per reaction of the bead mixture are transferred into a 96-well HybAid plate. Seven µL of 15 nM biotinylated detection oligonucleotide stock in 1× TE buffer are added to each reaction and mixed. Ten µL of neutralized assay sample are added and the plate is sealed with a silicon cap mat seal. The plate is first incubated at 96 °C for 5 minutes and incubated at 50°C without agitation overnight in a conventional hybridization oven. A filter plate (Dura pore, Millipore part number MSBVN1250, 1.2 µm pore size) is prewetted with 75 µL 1× TMAC hybridization solution supplemented with 0.5% (w/v) BSA. The entire sample volume from the hybridization reaction is transferred to the filter plate. The hybridization plate is rinsed with 75 µL 1× TMAC hybridization solution containing 0.5% BSA and any remaining material is transferred to the filter plate. Samples are filtered under slow vacuum, with 150 µL buffer evacuated over about 8 seconds. The filter plate is washed once with 75 µL 1× TMAC hybridization solution containing 0.5% BSA and the microspheres in the filter plate are resuspended in 75 µL 1× TMAC hybridization solution containing 0.5% BSA. The filter plate is protected from light and incubated on an Eppendorf Thermalmixer R for 5 minutes at 1000 rpm. The filter plate is then washed once with 75 µL 1× TMAC hybridization solution containing 0.5% BSA. 75 µL of 10 µg/mL streptavidin phycoerythrin (SAPE-100, MOSS, Inc.) in 1× TMAC hybridization solution is added to each reaction and incubated on Eppendorf Thermalmixer R at 25 °C at 1000 rpm for 60 minutes. The filter plate is washed twice with 75 µL 1× TMAC hybridization solution containing 0.5% BSA and the microspheres in the filter plate are resuspended in 75 µL 1× TMAC hybridization solution containing 0.5% BSA. The filter plate is then incubated protected from light on an Eppendorf Thermalmixer R for 5 minutes, 1000 rpm. The filter plate is then washed once with 75 µL 1× TMAC hybridization solution containing 0.5% BSA. Microspheres are resuspended in 75 µL 1× TMAC hybridization solution supplemented with 0.5% BSA, and analyzed on a Luminex 100 instrument running Xponent 3.0 software. At least 100 microspheres are counted per bead type, under high PMT calibration and a doublet discriminator setting of 7500 to 18000.

### QPCR read-out

Standard curves for qPCR are prepared in water ranging from 108 to 102 copies with 10-fold dilutions and a no-template control. Neutralized assay samples are diluted 40-fold into diH2O. The qPCR master mix is prepared at 2× final concentration (2× KOD buffer, 400 µM dNTP mix, 400 nM forward and reverse primer mix, 2× SYBR Green I and 0.5 U KOD EX). Ten µL of 2× qPCR master mix is added to 10 µL of diluted assay sample. qPCR is run on a BioRad MyIQ iCycler with 2 minutes at 96 °C followed by 40 cycles of 96 °C for 5 seconds and 72 °C for 30 seconds.

### Example 2. Fed-Fasted Plasma Model

In order to assess the duration of fasting before a blood draw (sample collection), a linear regression elastic net model containing a panel of the 7 biomarker proteins listed in Table 1 was developed. The model provides a predicted value that is the length of time, in hours, it has been since the subject's last meal. The training and verification datasets were obtained from analysis of samples from adult human volunteers, as described in Model Development below. Table 2 shows the performance metrics for the model. "CCC" is Concordance Correlation Coefficient. R-squared ("R²") is the degree of linear correlation, or the goodness-of-fit. "CI" is confidence interval. CCC and R² are indicative of predictive performance of the model. An R² of 1.0 (100%) indicates a perfect fit. Any r-squared value below 0.5 (50%) is considered poor correlation with regard to the specificity of the test. A panel of N biomarker proteins having a R² value of at least 0.600, at least 0.650, at least 0.700, at least 0.750, at least 0.800, at least 0.850, at least 0.900, at least 0.950 may be used to predict the fasting time.

**Table 2**

| **Model** | **Dataset** | **# of Analytes** | **Predictive Performance** | |
|---|---|---|---|---|
| | | | **CCC (95% Conf)** | **R²(95% Conf)** |
| Fed-fasted (plasma) | Training | 7 | 0.949 (0.915, 0.962) | 0.910 (0.852, 0.932) |
| | Verification | | 0.791 (0.748, 0.821) | 0.713 (0.599, 0.728) |

### Model Development

Twelve donors, each with four collection time-points were used to give a total of 48 samples. The goal of the fed-fasted study is to assess whether fasting before a blood draw impacts the plasma proteome. Donors fasted for at least 12 hours prior to the initial (fasted) blood collection, after which a standard moderate fat meal was consumed and subsequent blood draws at half hour to one-hour intervals was collected.

At each of four different timepoints (12 hours fasted, 0.5, 1 and 3 hrs post meal), a single K2EDTA Plasma tube (purple-topped, BD#367863) of blood was collected from each of twelve adult human volunteers by applying a tourniquet and using a 21G butterfly needle set. The tubes were inverted 8X-10x after collection and immediately centrifuged at 2,200xg for 15 minutes in a Beckman Coulter Allegra 25R centrifuge. The resulting plasma was carefully aspirated from each tube and stored as 0.75mL aliquots at - 80°C for 3 days. After short-term storage at -80°C, samples were thawed and 90µL aliquots transferred to matrix tubes for storage, again at -80°C, until the day that analysis with the aptamers was performed, for example, according to a protocol described in Example 1.

For this test, the data were split 83.3%/16.7% into training/verification datasets such that 2 samples were randomly selected from each of the 4 timepoints for the verification set (8 samples in the verification set) and the rest of the samples for the training set (40 samples in the training).

Due to low sample numbers, a hold-out test dataset was not used for model development. The model was developed on the training dataset and then assessed on the verification set, but it was not used as a holdout validation or test set. The sample numbers are shown in Table 3 below.

**Table 3: Sample numbers for fed-fasted plasma analysis**

| **Dataset** | **Total Samples** | **Number of Hours Since Meal** | | | |
|---|---|---|---|---|---|
| | | **0.5** | **1** | **3** | **12** |
| Training | 40 | 10 | 10 | 10 | 10 |
| Verification | 8 | 2 | 2 | 2 | 2 |

After the aptamer assay was performed, the data was normalized. Control samples were internally median normalized, plate scaled, calibrated, and samples normalized using adaptive normalization by maximum likelihood (ANML; see WO2021021678). All samples were within normalization scale factor pass/fail criteria and subsequently, no samples were removed from analysis. Normalization scale factors were not significantly correlated with time fasted.

### POC Results

The Proof of Concept (POC) results showed that a number of analytes were significant at different false-discovery rate (FDR) levels. Table 4 below shows those numbers and percentages (after removal of redlisted analytes) of analytes significant at different Type I error cut-offs for fed-fasted plasma results using a Wald test of the linear model regression coefficient.

**Table 4: Number and percentage of analytes significant at different Type I error cut-offs for fed-fasted plasma results**

| **FDR** | **# analytes (%) < FDR level** |
|---|---|
| 0.10 | 7 (0.09%) |
| 0.05 | 5 (0.07%) |
| 0.01 | 5 (0.07%) |

### Refinement and Validation

For the models developed in refinement, the training and verification sets described above were used.

Refinement focused on reducing the number of features without observing significant performance loss in the model, the reproducibility of predictions from replicate samples, and the orthogonality to other sample handling studies. Feature selection was performed by starting with the 39 features in an initial elastic net model from POC. Results from a best subset analysis showed that model performance plateaus with 10 or more features and that 5 or more features are used to achieve an adjusted R² of 0.95.

Thus, a series of elastic net models were generated with the number of features ranging from 5 to 15 analytes with 5 repeats of 10-fold cross-validation. Further selection was performed by testing performance on the other sample handling studies. The final model chosen had the best tradeoff with performance on the training set and low coefficient of variation (CV) with the other sample handling studies to ensure fasted predictions are independent of other sample handling conditions. Model predictions were tested in the training and verification data sets and calculated Lin's correlation coefficient for the training and verification data (Table 5).

The model performs well on the validation set with an R² of 0.713 and a root mean square error (RMSE) of 2.488 (Table 5).

**Table 5: Performance metrics for the fed-fasted plasma regression model**

| **Data set** | **R² (95% CI)** | **CCC (95% CI)** | **RMSE (95% CI)** |
|---|---|---|---|
| Training | 0.91 (0.852, 0.932) | 0.949 (0.915, 0.962) | 1.394 (1.303, 1.692) |
| Verification | 0.713 (0.599, 0.728) | 0.791 (0.748, 0.821) | 2.488 (1.868, 3.286) |

The effect of winsorization and dropping features were evaluated to correct for outlier values in other datasets. For the original data the RMSE was 2.612, with winsororization it was 4.227 and for feature removal it was 3.037. Outlier values were therefore replaced with zero for this model. These results are shown in Tables 6 and 7 below.

**Table 6: RMSE Values**

| **Model** | | **RMSE** | | **Final Selection** |
|---|---|---|---|---|
| | **Original (training)** | **Winsorized** | **Replacement with Zero** | |
| Fed-fasted (plasma) | 2.612 | 4.227 | 3.037 | Replacement with zero |

**Table 7: Predictive variation due to assay noise**

| **Model** | **Bound** | **Error Bounds (95%)** | **Error Bounds (99%)** |
|---|---|---|---|
| Fed-fasted (plasma) | Upper Bound | 0.838 | 1.087 |
| | Lower Bound | -0.826 | -1.195 |

### Example 3: Use of the Sample Handling Model

The sample handling model may be used to censor individual samples for specific output that is important to the test. In one embodiment, samples that are identified as failing the quality assessment for the fasting time may be removed if the specific output of the fasting time is important to the test being conducted. In other embodiments, samples that are identified as failing the quality assessment for the fasting time may be included if the specific output of the fasting time is not important to the test being conducted. In some embodiments, a panel of biomarker proteins may be modified in a subsequent or simultaneous analysis such as protein biomarker discovery analysis, protein expression level analysis, diagnostic method or prognostic method based on the determined approximate times for each of the plurality of samples. In some embodiments, the panel of biomarker proteins is reduced in number of biomarker proteins measured.

The sample handling model may be used to identify biases within a plurality of samples collected. In one embodiment, the sample handling model may be used to identify biases between experimental samples and controls.

The sample handling model may be used to assess adherence to a clinical study protocol for sample collection and processing.

The sample handling model may be used to identify outlier samples in a plurality of samples. In one embodiment, the outlier sample may be 1 or 2 or 3 or more standard deviations from the other samples in the model. The outlier sample may have better or worse quality relative to the other samples in the model.

The sampling model may be used to compare a plurality of samples from a first site to one or more additional sites for sample collection and processing.

### Example 4: Analysis of Fed-Fasted Biomarker Panel Model

Model biomarker panels comprising various combinations of the biomarkers listed in Table 1 were analyzed to determine the coefficient of determination (R²) value for the various combinations. Tables 8-13 below shows the model results when various combinations comprising 1 to 7 biomarker proteins were measured. The results are shown in Tables 8-13. that the performance of panels comprising at least one biomarker from the biomarkers listed in Table 1 are provided.

**Table 8: Performance of panels comprising1 biomarker**

| **GCG** | **GIP** | **FABP1** | **FGFBP1** | **INS** | **PPY** | **TMED10** | **Train RMSE** | **TrainRs quared** | **Train MAE** | **TestR MSE** | **TestRs quared** | **SampleHan dling_CV** |
|---|---|---|---|---|---|---|---|---|---|---|---|---|
| GCG | | | | | | | 2.909 | 0.697 | 2.368 | 4.13 | 0.221 | 0.202 |
| | GIP | | | | | | 2.704 | 0.708 | 2.244 | 3.69 | 0.394 | 0.203 |
| | | FABP1 | | | | | 4.146 | 0.484 | 3.432 | 4.07 | 0.477 | 0.21 |
| | | | FGFBP1 | | | | 3.191 | 0.667 | 2.571 | 3.28 | 0.686 | 0.313 |
| | | | | INS | | | 3.458 | 0.492 | 3.034 | 3.17 | 0.581 | 0.181 |
| | | | | | PPY | | 3.985 | 0.416 | 3.325 | 3.01 | 0.64 | 0.086 |
| | | | | | | TMED10 | 4.494 | 0.346 | 3.679 | 4.5 | 0.089 | 0.215 |

**Table 9: Performance of panels comprising 2 biomarkers**

| **GCG** | **GIP** | **FABP1** | **FGFBP1** | **INS** | **PPY** | **TMED10** | **TrainR MSE** | **TrainRs quared** | **Train MAE** | **TestR MSE** | **TestRs quared** | **SampleHand ling_CV** |
|---|---|---|---|---|---|---|---|---|---|---|---|---|
| GCG | GIP | | | | | | 2.371 | 0.8 | 1.873 | 3.91 | 0.299 | 0.202 |
| GCG | | FABP1 | | | | | 2.715 | 0.751 | 2.321 | 3.81 | 0.378 | 0.222 |
| GCG | | | FGFBP1 | | | | 2.474 | 0.793 | 2.023 | 3.58 | 0.465 | 0.227 |
| GCG | | | | INS | | | 2.574 | 0.745 | 2.134 | 3.39 | 0.49 | 0.196 |
| GCG | | | | | PPY | | 2.783 | 0.7 | 2.212 | 3.34 | 0.543 | 0.182 |
| GCG | | | | | | TMED10 | 2.915 | 0.676 | 2.302 | 3.96 | 0.308 | 0.203 |
| | GIP | FABP1 | | | | | 2.46 | 0.794 | 2.008 | 3.43 | 0.582 | 0.215 |
| | GIP | | FGFBP1 | | | | 2.546 | 0.776 | 2.036 | 3.4 | 0.551 | 0.218 |
| | GIP | | | INS | | | 2.709 | 0.729 | 2.337 | 3.31 | 0.555 | 0.16 |
| | GIP | | | | PPY | | 2.371 | 0.8 | 1.927 | 2.86 | 0.675 | 0.165 |
| | GIP | | | | | TMED10 | 2.709 | 0.726 | 2.249 | 3.57 | 0.485 | 0.218 |
| | | FABP1 | FGFBP1 | | | | 2.997 | 0.778 | 2.426 | 3.19 | 0.795 | 0.31 |
| | | FABP1 | | INS | | | 3.083 | 0.703 | 2.574 | 2.82 | 0.809 | 0.197 |
| | | FABP1 | | | PPY | | 3.597 | 0.572 | 3.028 | 3.05 | 0.675 | 0.138 |
| | | FABP1 | | | | TMED10 | 4.034 | 0.562 | 3.269 | 4.06 | 0.283 | 0.235 |
| | | | FGFBP1 | INS | | | 2.783 | 0.738 | 2.325 | 2.78 | 0.746 | 0.234 |
| | | | FGFBP1 | | PPY | | 2.844 | 0.715 | 2.321 | 2.64 | 0.804 | 0.282 |
| | | | FGFBP1 | | | TMED10 | 3.174 | 0.647 | 2.527 | 3.41 | 0.615 | 0.324 |
| | | | | INS | PPY | | 3.14 | 0.611 | 2.675 | 2.25 | 0.83 | 0.162 |
| | | | | INS | | TMED10 | 3.377 | 0.527 | 2.908 | 3.28 | 0.525 | 0.218 |
| | | | | | PPY | TMED10 | 4.036 | 0.37 | 3.355 | 3.24 | 0.594 | 0.116 |

**Table 10: Performance of panels comprising 3 biomarkers**

| **GCG** | **GIP** | **FABP1** | **FGFBP1** | **INS** | **PPY** | **TMED10** | **TrainR MSE** | **TrainRs quared** | **Train MAE** | **TestR MSE** | **TestRs quared** | **SampleHand ling CV** |
|---|---|---|---|---|---|---|---|---|---|---|---|---|
| GCG | GIP | FABP1 | | | | | 2.123 | 0.849 | 1.735 | 3.61 | 0.429 | 0.213 |
| GCG | GIP | | FGFBP1 | | | | 2.243 | 0.84 | 1.826 | 3.59 | 0.43 | 0.229 |
| GCG | GIP | | | INS | | | 2.292 | 0.815 | 1.873 | 3.52 | 0.436 | 0.188 |
| GCG | GIP | | | | PPY | | 2.139 | 0.857 | 1.662 | 3.11 | 0.588 | 0.185 |
| GCG | GIP | | | | | TMED10 | 2.338 | 0.813 | 1.844 | 3.7 | 0.392 | 0.208 |
| GCG | | FABP1 | FGFBP1 | | | | 2.196 | 0.853 | 1.814 | 3.35 | 0.588 | 0.26 |
| GCG | | FABP1 | | INS | | | 2.204 | 0.846 | 1.919 | 3.05 | 0.636 | 0.216 |
| GCG | | FABP1 | | | PPY | | 2.555 | 0.764 | 2.199 | 3.14 | 0.654 | 0.189 |
| GCG | | FABP1 | | | | TMED10 | 2.771 | 0.711 | 2.292 | 3.67 | 0.461 | 0.231 |
| GCG | | | FGFBP1 | INS | | | 2.244 | 0.822 | 1.837 | 3.14 | 0.589 | 0.2 |
| GCG | | | FGFBP1 | | PPY | | 2.327 | 0.818 | 1.938 | 2.95 | 0.694 | 0.207 |
| GCG | | | FGFBP1 | | | TMED10 | 2.479 | 0.774 | 2.034 | 3.44 | 0.544 | 0.239 |
| GCG | | | | INS | PPY | | 2.419 | 0.754 | 1.895 | 2.77 | 0.695 | 0.189 |
| GCG | | | | INS | | TMED10 | 2.538 | 0.737 | 2.047 | 3.24 | 0.55 | 0.207 |
| GCG | | | | | PPY | TMED10 | 2.808 | 0.676 | 2.242 | 3.28 | 0.593 | 0.188 |
| | GIP | FABP1 | FGFBP1 | | | | 2.294 | 0.844 | 1.818 | 3.15 | 0.69 | 0.263 |
| | GIP | FABP1 | | INS | | | 2.425 | 0.829 | 2.07 | 2.94 | 0.729 | 0.186 |
| | GIP | FABP1 | | | PPY | | 2.085 | 0.849 | 1.738 | 2.69 | 0.749 | 0.182 |
| | GIP | FABP 1 | | | | TMED10 | 2.481 | 0.782 | 2.016 | 3.31 | 0.633 | 0.236 |
| | GIP | | FGFBP1 | INS | | | 2.515 | 0.786 | 2.137 | 3.02 | 0.663 | 0.2 |
| | GIP | | FGFBP1 | | PPY | | 2.202 | 0.843 | 1.824 | 2.68 | 0.741 | 0.191 |
| | GIP | | FGFBP1 | | | TMED10 | 2.513 | 0.783 | 2.07 | 3.27 | 0.613 | 0.282 |
| | GIP | | | INS | PPY | | 2.343 | 0.796 | 1.932 | 2.54 | 0.753 | 0.161 |
| | GIP | | | INS | | TMED10 | 2.625 | 0.742 | 2.325 | 3.16 | 0.607 | 0.204 |
| | GIP | | | | PPY | TMED10 | 2.349 | 0.801 | 1.9 | 2.8 | 0.717 | 0.174 |
| | | FABP1 | FGFBP1 | INS | | | 2.585 | 0.835 | 2.08 | 2.51 | 0.858 | 0.28 |
| | | FABP1 | FGFBP1 | | PPY | | 2.669 | 0.788 | 2.169 | 2.54 | 0.852 | 0.298 |
| | | FABP1 | FGFBP1 | | | TMED10 | 3.026 | 0.75 | 2.382 | 3.21 | 0.716 | 0.334 |
| | | FABP1 | | INS | PPY | | 2.829 | 0.73 | 2.426 | 2.02 | 0.903 | 0.175 |
| | | FABP1 | | INS | | TMED10 | 3.044 | 0.687 | 2.529 | 2.89 | 0.677 | 0.231 |
| | | FABP1 | | | PPY | TMED10 | 3.706 | 0.544 | 3.066 | 3.09 | 0.661 | 0.163 |
| | | | FGFBP1 | INS | PPY | | 2.533 | 0.775 | 2.14 | 2.25 | 0.825 | 0.223 |
| | | | FGFBP1 | INS | | TMED10 | 2.714 | 0.724 | 2.217 | 2.78 | 0.736 | 0.324 |
| | | | FGFBP1 | | PPY | TMED10 | 2.91 | 0.698 | 2.382 | 2.68 | 0.806 | 0.303 |
| | | | | INS | PPY | TMED10 | 3.169 | 0.596 | 2.768 | 2.35 | 0.798 | 0.181 |

**Table 11: Performance of panels comprising 4 biomarkers**

| **GCG** | **GIP** | **FABP1** | **FGFBP1** | **INS** | **PPY** | **TMED10** | **TrainR MSE** | **TrainRs quared** | **Train MAE** | **TestR MSE** | **TestRs quared** | **SampleHand ling_CV** |
|---|---|---|---|---|---|---|---|---|---|---|---|---|
| GCG | GIP | FABP1 | FGFBP1 | | | | 1.945 | 0.883 | 1.633 | 3.36 | 0.542 | 0.252 |
| GCG | GIP | FABP1 | | INS | | | 1.951 | 0.882 | 1.644 | 3.2 | 0.569 | 0.202 |
| GCG | GIP | FABP1 | | | PPY | | 1.869 | 0.885 | 1.523 | 2.94 | 0.667 | 0.195 |
| GCG | GIP | FABP1 | | | | TMED10 | 2.159 | 0.836 | 1.759 | 3.46 | 0.506 | 0.225 |
| GCG | GIP | | FGFBP1 | INS | | | 2.185 | 0.845 | 1.809 | 3.29 | 0.528 | 0.201 |
| GCG | GIP | | FGFBP1 | | PPY | | 2.022 | 0.882 | 1.641 | 2.92 | 0.662 | 0.203 |
| GCG | GIP | | FGFBP1 | | | TMED10 | 2.223 | 0.843 | 1.832 | 3.43 | 0.511 | 0.238 |
| GCG | GIP | | | INS | PPY | | 2.101 | 0.849 | 1.677 | 2.86 | 0.658 | 0.187 |
| GCG | GIP | | | INS | | TMED10 | 2.262 | 0.825 | 1.899 | 3.34 | 0.513 | 0.198 |
| GCG | GIP | | | | PPY | TMED10 | 2.134 | 0.859 | 1.657 | 3.04 | 0.634 | 0.19 |
| GCG | | FABP1 | FGFBP1 | INS | | | 1.876 | 0.895 | 1.601 | 2.89 | 0.696 | 0.255 |
| GCG | | FABP1 | FGFBP1 | | PPY | | 2.055 | 0.86 | 1.75 | 2.81 | 0.759 | 0.254 |
| GCG | | FABP1 | FGFBP1 | | | TMED10 | 2.301 | 0.815 | 1.877 | 3.26 | 0.641 | 0.281 |
| GCG | | FABP1 | | INS | PPY | | 2.058 | 0.84 | 1.729 | 2.56 | 0.775 | 0.205 |
| GCG | | FABP1 | | INS | | TMED10 | 2.225 | 0.815 | 1.82 | 2.98 | 0.663 | 0.227 |
| GCG | | FABP1 | | | PPY | TMED10 | 2.669 | 0.721 | 2.261 | 3.11 | 0.682 | 0.197 |
| GCG | | | FGFBP1 | INS | PPY | | 2.099 | 0.838 | 1.67 | 2.61 | 0.747 | 0.204 |
| GCG | | | FGFBP1 | INS | | TMED10 | 2.21 | 0.813 | 1.805 | 3.03 | 0.638 | 0.237 |
| GCG | | | FGFBP1 | | PPY | TMED10 | 2.375 | 0.795 | 1.998 | 2.91 | 0.729 | 0.233 |
| GCG | | | | INS | PPY | TMED10 | 2.446 | 0.743 | 1.919 | 2.73 | 0.716 | 0.195 |
| | GIP | FABP1 | FGFBP1 | INS | | | 2.286 | 0.864 | 1.908 | 2.73 | 0.789 | 0.239 |
| | GIP | FABP1 | FGFBP1 | | PPY | | 1.941 | 0.884 | 1.648 | 2.54 | 0.801 | 0.211 |
| | GIP | FABP1 | FGFBP1 | | | TMED10 | 2.326 | 0.828 | 1.867 | 3.08 | 0.716 | 0.307 |
| | GIP | FABP1 | | INS | PPY | | 2.08 | 0.857 | 1.722 | 2.33 | 0.83 | 0.174 |
| | GIP | FABP1 | | INS | | TMED10 | 2.406 | 0.813 | 2.032 | 2.87 | 0.73 | 0.211 |
| | GIP | FABP1 | | | PPY | TMED10 | 2.117 | 0.844 | 1.786 | 2.66 | 0.777 | 0.185 |
| | GIP | | FGFBP1 | INS | PPY | | 2.19 | 0.837 | 1.826 | 2.47 | 0.771 | 0.168 |
| | GIP | | FGFBP1 | INS | | TMED10 | 2.439 | 0.789 | 2.105 | 2.92 | 0.703 | 0.245 |
| | GIP | | FGFBP1 | | PPY | TMED10 | 2.179 | 0.845 | 1.828 | 2.64 | 0.772 | 0.22 |
| | GIP | | | INS | PPY | TMED10 | 2.32 | 0.795 | 1.89 | 2.48 | 0.786 | 0.167 |
| | | FABP1 | FGFBP1 | INS | PPY | | 2.357 | 0.847 | 1.916 | 2.08 | 0.882 | 0.244 |
| | | FABP1 | FGFBP1 | INS | | TMED10 | 2.568 | 0.807 | 2.016 | 2.55 | 0.819 | 0.33 |
| | | FABP1 | FGFBP1 | | PPY | TMED10 | 2.788 | 0.755 | 2.278 | 2.59 | 0.845 | 0.32 |
| | | FABP1 | | INS | PPY | TMED10 | 2.903 | 0.703 | 2.51 | 2.14 | 0.86 | 0.187 |
| | | | FGFBP1 | INS | PPY | TMED10 | 2.551 | 0.766 | 2.127 | 2.28 | 0.822 | 0.267 |

**Table 12: Performance of panels comprising 5 biomarkers**

| **GCG** | **GIP** | **FABP1** | **FGFBP1** | **INS** | **PPY** | **TMED10** | **TrainR MSE** | **TrainRs quared** | **Train MAE** | **TestR MSE** | **TestRs quared** | **SampleHand ling_CV** |
|---|---|---|---|---|---|---|---|---|---|---|---|---|
| GCG | GIP | FABP1 | FGFBP1 | INS | | | 1.815 | 0.904 | 1.535 | 3.03 | 0.638 | 0.23 |
| GCG | GIP | FABP1 | FGFBP1 | | PPY | | 1.722 | 0.908 | 1.492 | 2.78 | 0.727 | 0.23 |
| GCG | GIP | FABP1 | FGFBP1 | | | TMED10 | 2.02 | 0.864 | 1.686 | 3.24 | 0.604 | 0.272 |
| GCG | GIP | FABP1 | | INS | PPY | | 1.735 | 0.896 | 1.405 | 2.66 | 0.735 | 0.189 |
| GCG | GIP | FABP1 | | INS | | TMED10 | 1.96 | 0.868 | 1.583 | 3.08 | 0.622 | 0.21 |
| GCG | GIP | FABP1 | | | PPY | TMED10 | 1.918 | 0.874 | 1.562 | 2.89 | 0.701 | 0.197 |
| GCG | GIP | | FGFBP1 | INS | PPY | | 1.991 | 0.874 | 1.611 | 2.72 | 0.707 | 0.195 |
| GCG | GIP | | FGFBP1 | INS | | TMED10 | 2.14 | 0.846 | 1.786 | 3.15 | 0.59 | 0.224 |
| GCG | GIP | | FGFBP1 | | PPY | TMED10 | 2.013 | 0.883 | 1.642 | 2.87 | 0.699 | 0.217 |
| GCG | GIP | | | INS | PPY | TMED10 | 2.098 | 0.849 | 1.698 | 2.79 | 0.693 | 0.192 |
| GCG | | FABP1 | FGFBP1 | INS | PPY | | 1.734 | 0.895 | 1.457 | 2.45 | 0.809 | 0.239 |
| GCG | | FABP1 | FGFBP1 | INS | | TMED10 | 1.917 | 0.867 | 1.617 | 2.82 | 0.722 | 0.273 |
| GCG | | FABP1 | FGFBP1 | | PPY | TMED10 | 2.207 | 0.823 | 1.858 | 2.8 | 0.781 | 0.268 |
| GCG | | FABP1 | | INS | PPY | TMED10 | 2.143 | 0.812 | 1.735 | 2.55 | 0.784 | 0.21 |
| GCG | | | FGFBP1 | INS | PPY | TMED10 | 2.116 | 0.829 | 1.705 | 2.59 | 0.764 | 0.224 |
| | GIP | FABP1 | FGFBP1 | INS | PPY | | 1.956 | 0.889 | 1.626 | 2.31 | 0.829 | 0.195 |
| | GIP | FABP1 | FGFBP1 | INS | | TMED10 | 2.27 | 0.846 | 1.9 | 2.69 | 0.794 | 0.266 |
| | GIP | FABP1 | FGFBP1 | | PPY | TMED10 | 1.992 | 0.877 | 1.707 | 2.53 | 0.82 | 0.236 |
| | GIP | FABP1 | | INS | PPY | TMED10 | 2.109 | 0.849 | 1.755 | 2.3 | 0.848 | 0.177 |
| | GIP | | FGFBP1 | INS | PPY | TMED10 | 2.161 | 0.837 | 1.814 | 2.45 | 0.787 | 0.197 |
| | | FABP1 | FGFBP1 | INS | PPY | TMED10 | 2.422 | 0.824 | 1.953 | 2.13 | 0.872 | 0.281 |

**Table 13: Performance of panels comprising 6 and 7 biomarkers**

| **GCG** | **GIP** | **FABP1** | **FGFBP1** | **INS** | **PPY** | **TMED10** | **TrainR MSE** | **TrainRs quared** | **Train MAE** | **TestR MSE** | **TestRs quared** | **SampleHand ling_CV** |
|---|---|---|---|---|---|---|---|---|---|---|---|---|
| GCG | GIP | FABP1 | FGFBP1 | INS | PPY | | 1.62 | 0.915 | 1.38 | 2.55 | 0.771 | 0.213 |
| GCG | GIP | FABP1 | FGFBP1 | INS | | TMED10 | 1.848 | 0.885 | 1.565 | 2.94 | 0.679 | 0.244 |
| GCG | GIP | FABP1 | FGFBP1 | | PPY | TMED10 | 1.807 | 0.895 | 1.566 | 2.75 | 0.753 | 0.242 |
| GCG | GIP | FABP1 | | INS | PPY | TMED10 | 1.787 | 0.884 | 1.418 | 2.62 | 0.758 | 0.194 |
| GCG | GIP | | FGFBP1 | INS | PPY | TMED10 | 1.975 | 0.874 | 1.635 | 2.67 | 0.734 | 0.213 |
| GCG | | FABP1 | FGFBP1 | INS | PPY | TMED10 | 1.835 | 0.87 | 1.528 | 2.45 | 0.817 | 0.25 |
| | GIP | FABP1 | FGFBP1 | INS | PPY | TMED10 | 1.995 | 0.88 | 1.677 | 2.29 | 0.842 | 0.219 |
| GCG | GIP | FABP1 | FGFBP1 | INS | PPY | TMED10 | 1.692 | 0.902 | 1.42 | 2.53 | 0.789 | 0.222 |

## Claims

1. A method of assessing quality of a sample collected from a subject comprising detecting the level of each of N biomarker proteins in the sample, wherein N is at least 2, and wherein one of the biomarker proteins is Fibroblast growth factor-binding protein 1 (FGFP1), and at least one of the N biomarker proteins is selected from Transmembrane emp24 domaincontaining protein 10 (TMEDA), Glucagon, Gastric inhibitory polypeptide (GIP), Fatty acidbinding protein, liver (FABPL), Insulin, and Pancreatic hormone (PH), wherein the sample is a plasma sample, and wherein the detecting is performed to determine the approximate fasting time that elapsed from a meal to sample collection.

2. The method of claim 1, wherein 1 of the N biomarker proteins is TMEDA; or
wherein 1 of the N biomarker proteins is Glucagon; or
wherein 1 of the N biomarker proteins is GIP; or
wherein 1 of the N biomarker proteins is FABPL; or
wherein 1 of the N biomarker proteins is Insulin; or
wherein 1 of the N biomarker proteins is PH; or
wherein 2 of the N biomarker proteins are TMEDA and Glucagon; or
wherein 2 of the N biomarker proteins are TMEDA and GIP; or
wherein 2 of the N biomarker proteins are TMEDA and FABPL; or
wherein 2 of the N biomarker proteins are TMEDA and Insulin; or
wherein 2 of the N biomarker proteins are TMEDA and PH; or
wherein 2 of the N biomarker proteins are Glucagon and GIP; or
wherein 2 of the N biomarker proteins are Glucagon and FABPL; or
wherein 2 of the N biomarker proteins are Glucagon and Insulin; or
wherein 2 of the N biomarker proteins are Glucagon and PH; or
wherein 2 of the N biomarker proteins are GIP and FABPL; or
wherein 2 of the N biomarker proteins are GIP and Insulin; or
wherein 2 of the N biomarker proteins are GIP and PH; or
wherein 2 of the N biomarker proteins are FABPL and Insulin; or
wherein 2 of the N biomarker proteins are FABPL and PH; or
wherein 2 of the N biomarker proteins are Insulin and PH.

3. The method of claim 1 or 2, wherein N is 3, N is 4, N is 5, N is 6, N is 7, optionally wherein all of the N biomarker proteins are selected from FGFP1, TMEDA, Glucagon, GIP, FABPL, Insulin, and PH.

4. The method of any one of claims 1-3, wherein the sample was processed, frozen, and thawed after the sample collection and prior to the detecting.

5. The method of claim 4, wherein the determining is based on comparing the detected levels of the N biomarker proteins to reference levels, wherein the reference levels are average levels of the N biomarker proteins present in samples having fasting times of 0.5, 1, 3, or 12 hours, optionally wherein the detected levels of each of the N biomarker proteins compared to the reference levels indicate that the approximate time that elapsed from meal to sample collection was greater than 0.5, greater than 1, greater than 2, greater than 3, greater than 4, greater than 5, greater than 6, greater than 7, greater than 8, greater than 9, greater than 10, greater than 11, or greater than 12 hours.

6. The method of claim 4 or 5, wherein the determining is based on a panel of N biomarker proteins having an R² value of at least 0.600, at least 0.650, at least 0.700, at least 0.750, at least 0.800, at least 0.850, at least 0.900, or at least 0.950.

7. The method of any one of claims 4-6, comprising performing protein biomarker discovery analysis, protein expression level analysis, a diagnostic method, or a prognostic method on the sample.

8. The method of any one of claims 1-7, comprising identifying the sample as passing a quality assessment or failing a quality assessment, wherein the identifying is based, at least in part, on the detected levels of the N biomarker proteins.

9. The method of claim 8, wherein the sample is identified as passing if the approximate fasting time that elapsed from meal until sample collection is determined to be greater than 3 hours, greater than 4 hours, greater than 5 hours, greater than 6 hours, greater than 7 hours, greater than 8 hours, greater than 9 hours, greater than 10 hours, greater than 11 hours, greater than 12 hours, greater than 13 hours, greater than 14 hours, greater than 15 hours, greater than 16 hours, greater than 17 hours, greater than 18 hours, greater than 19 hours, greater than 20 hours, greater than 21 hours, greater than 22 hours, greater than 23 hours, or greater than 24 hours.

10. The method of any one of claims 1-9, wherein the detecting comprises performing mass spectrometry, an aptamer based assay, and/or an antibody based assay.

11. The method of any one of claims 1-10, wherein the method comprises contacting biomarker proteins of the sample from the subject with a set of capture reagents, wherein each capture reagent of the set of capture reagents specifically binds to one biomarker protein being detected, optionally, wherein each the capture reagents specifically binds to a different biomarker protein being detected.

12. The method of claim 11, wherein each capture reagent is an antibody or an aptamer.

13. The method of claim 12, wherein each capture reagent is an aptamer.

14. The method of claim 13, wherein at least one aptamer is a slow off-rate aptamer, optionally, wherein each slow off-rate aptamer binds to its target protein with an off rate (t_{½}) of ≥ 30 minutes, ≥ 60 minutes, ≥ 90 minutes, ≥ 120 minutes, ≥ 150 minutes, ≥ 180 minutes, ≥ 210 minutes, or ≥ 240 minutes, and/or optionally, wherein at least one slow off-rate aptamer comprises at least 1, at least 2, at least 3, at least 4, at least 5, at least 6, at least 7, at least 8, at least 9, or at least 10 nucleotides with modifications.

## Patentansprüche

1. Verfahren zur Beurteilung der Qualität einer Probe, die von einem Individuum entnommen wurde, welches das Detektieren des Spiegels jedes der N Biomarker-Proteine in der Probe umfasst, wobei N zumindest 2 ist und wobei eines der Biomarker-Proteine das Fibroblasten-Wachstumsfaktor-Bindungsprotein 1 (FGFP1) ist und zumindest eines der N Biomarker-Proteine aus dem Transmembran-emp24-Domäne-hältigen Protein 10 (TMEDA), Glucagon, dem gastroinhibitorischen Polypeptid (GIP), dem Fettsäure bindenden Protein, Leber (FABPL), Insulin und dem Pankreashormon (PH) ausgewählt ist, wobei die Probe eine Plasmaprobe ist und wobei das Detektieren durchgeführt wird, um die ungefähre Nahrungskarenzdauer zu bestimmen, die von einer Mahlzeit bis zur Probenentnahme vergangen ist.

2. Verfahren nach Anspruch 1, wobei eines der N Biomarker-Proteine TMEDA ist; oder
wobei eines der N Biomarker-Proteine Glucagon ist; oder
wobei eines der N Biomarker-Proteine GIP ist; oder
wobei eines der N Biomarker-Proteine FABPL ist; oder
wobei eines der N Biomarker-Proteine Insulin ist; oder
wobei eines der N Biomarker-Proteine PH ist; oder
wobei zwei der N Biomarker-Proteine TMEDA und Glucagon sind; oder
wobei zwei der N Biomarker-Proteine TMEDA und GIP sind; oder
wobei zwei der N Biomarker-Proteine TMEDA und FABPL sind; oder
wobei zwei der N Biomarker-Proteine TMEDA und Insulin sind; oder
wobei zwei der N Biomarker-Proteine TMEDA und PH sind; oder
wobei zwei der N Biomarker-Proteine Glucagon und GIP sind; oder
wobei zwei der N Biomarker-Proteine Glucagon und FABPL sind; oder
wobei zwei der N Biomarker-Proteine Glucagon und Insulin sind; oder
wobei zwei der N Biomarker-Proteine Glucagon und PH sind; oder
wobei zwei der N Biomarker-Proteine GIP und FABPL sind; oder
wobei zwei der N Biomarker-Proteine GIP und Insulin sind; oder
wobei zwei der N Biomarker-Proteine GIP und PH sind; oder
wobei zwei der N Biomarker-Proteine FABPL und Insulin sind; oder
wobei zwei der N Biomarker-Proteine FABPL und PH sind; oder
wobei zwei der N Biomarker-Proteine Insulin und PH sind.

3. Verfahren nach Anspruch 1 oder 2, wobei N = 3 ist, N = 4 ist, N = 5 ist, N = 6 ist, N = 7 ist, wobei gegebenenfalls jedes der N Biomarker-Proteine aus FGFP1, TMEDA, Glucagon, GIP, FABPL, Insulin und PH ausgewählt ist.

4. Verfahren nach einem der Ansprüche 1 bis 3, wobei die Probe nach der Probenentnahme und vor der Detektion verarbeitet, eingefroren und aufgetaut wurde.

5. Verfahren nach Anspruch 4, wobei das Bestimmen auf einem Vergleich zwischen der detektierten Spiegel der N Biomarker-Proteine mit Referenzspiegeln basiert, wobei die Referenzspiegel durchschnittliche Spiegel der N Biomarker-Proteine in Proben mit einer Nahrungskarenzdauer von 0,5, 1, 3 oder 12 Stunden sind, wobei gegebenenfalls die detektierten Spiegel der einzelnen N Biomarker-Proteine im Vergleich zu den Referenzspiegeln anzeigen, dass die ungefähre Dauer von der Mahlzeit bis zur Probenentnahme mehr als 0,5, mehr als 1, mehr als 2, mehr als 3, mehr als 4, mehr als 5, mehr als 6, mehr als 7, mehr als 8, mehr als 9, mehr als 10, mehr als 11 oder mehr als 12 Stunden betrug.

6. Verfahren nach einem der Ansprüche 4 oder 5, wobei das Bestimmen auf einem Panel von N Biomarker-Proteinen mit einem R²-Wert von zumindest 0,600, zumindest 0,650, zumindest 0,700, zumindest 0,750, zumindest 0,800, zumindest 0,850, zumindest 0,900 oder zumindest 0,950 basiert.

7. Verfahren nach einem der Ansprüche 4 bis 6, welches das Durchführen einer Protein-Biomarker-Entdeckungsanalyse, Proteinexpressionsspiegelanalyse, eines diagnostischen Verfahrens oder eines prognostischen Verfahrens an der Probe umfasst.

8. Verfahren nach einem der Ansprüche 1 bis 7, welches das Identifizieren der Probe als eine Qualitätsbeurteilung bestehend oder eine Qualitätsbeurteilung nicht bestehend umfasst, wobei das Identifizieren zumindest teilweise auf den detektierten Spiegeln der N Biomarker-Proteine basiert.

9. Verfahren nach Anspruch 8, wobei die Probe als bestehend identifiziert wird, wenn die ungefähre Nahrungskarenzdauer von der Mahlzeit bis zur Probenentnahme mit mehr als 3 Stunden, mehr als 4 Stunden, mehr als 5 Stunden, mehr als 6 Stunden, mehr als 7 Stunden, mehr als 8 Stunden, mehr als 9 Stunden, mehr als 10 Stunden, mehr als 11 Stunden, mehr als 12 Stunden, mehr als 13 Stunden, mehr als 14 Stunden, mehr als 15 Stunden, mehr als 16 Stunden, mehr als 17 Stunden, mehr als 18 Stunden, mehr als 19 Stunden, mehr als 20 Stunden, mehr als 21 Stunden, mehr als 22 Stunden, mehr als 23 Stunden oder mehr als 24 Stunden bestimmt wird.

10. Verfahren nach einem der Ansprüche 1 bis 9, wobei das Detektieren das Durchführen von Massenspektrometrie, eines Aptamer-basierten Tests und/oder eines Antikörper-basierten Tests umfasst.

11. Verfahren nach einem der Ansprüche 1 bis 10, wobei das Verfahren das In-Kontakt-Bringen von Biomarker-Proteinen der Probe vom Individuum mit einem Satz von Fängerreagenzien umfasst, wobei jedes Fängerreagens des Satzes von Fängerreagenzien spezifisch an ein Biomarker-Protein bindet, das detektiert wird, wobei gegebenenfalls jedes der Fängerreagenzien spezifisch an ein anderes Biomarker-Protein bindet, das detektiert wird.

12. Verfahren nach Anspruch 11, wobei jedes Fängerreagens ein Antikörper oder ein Aptamer ist.

13. Verfahren nach Anspruch 12, wobei jedes Fängerreagens ein Aptamer ist.

14. Verfahren nach Anspruch 13, wobei zumindest ein Aptamer ein Aptamer mit langsamer Dissoziationsrate ist, wobei jedes Aptamer mit langsamer Dissoziationsrate an sein Zielprotein mit einer Dissoziationsrate (t_{½}) von ≥ 30 Minuten, ≥ 60 Minuten, ≥ 90 Minuten, ≥ 120 Minuten, ≥ 150 Minuten, ≥ 180 Minuten, ≥ 210 Minuten oder ≥ 240 Minuten bindet und/oder wobei gegebenenfalls zumindest ein Aptamer mit langsamer Dissoziationsrate zumindest 1, zumindest 2, zumindest 3, zumindest 4, zumindest 5, zumindest 6, zumindest 7, zumindest 8, zumindest 9 oder zumindest 10 Nukleotide mit Modifikationen umfasst.

## Revendications

1. Procédé d'évaluation de la qualité d'un échantillon prélevé chez un sujet comprenant une détection du niveau de chacune des N protéines de biomarqueur dans l'échantillon, dans lequel N est au moins 2, et dans lequel l'une des protéines de biomarqueur est la protéine 1 de liaison au facteur de croissance des fibroblastes (FGFP1), et au moins l'une des N protéines de biomarqueur est sélectionnée parmi la protéine 10 contenant le domaine emp24 transmembranaire (TMEDA), le glucagon, le polypeptide inhibiteur gastrique (GIP), la protéine de liaison aux acides gras de type hépatique (FABPL), l'insuline et l'hormone pancréatique (PH), dans lequel l'échantillon est un échantillon de plasma, et dans lequel la détection est effectuée pour déterminer le temps de jeûne approximatif qui s'est écoulé entre un repas et le prélèvement d'échantillon.

2. Procédé selon la revendication 1, dans lequel 1 des N protéines de biomarqueur est TMEDA ; ou
dans lequel 1 des N protéines de biomarqueur est le glucagon ; ou
dans lequel 1 des N protéines de biomarqueur est GIP ; ou
dans lequel 1 des N protéines de biomarqueur est FABPL ; ou
dans lequel 1 des N protéines de biomarqueur est l'insuline ; ou
dans lequel 1 des N protéines de biomarqueur est PH ; ou
dans lequel 2 des N protéines de biomarqueur sont le TMEDA et le glucagon ; ou
dans lequel 2 des N protéines de biomarqueur sont TMEDA et GIP ; ou
dans lequel 2 des N protéines de biomarqueur sont TMEDA et FABPL ; ou
dans lequel 2 des N protéines de biomarqueur sont le TMEDA et l'insuline ; ou
dans lequel 2 des N protéines de biomarqueur sont TMEDA et PH ; ou
dans lequel 2 des N protéines de biomarqueur sont le glucagon et le GIP ; ou
dans lequel 2 des N protéines de biomarqueur sont le glucagon et le FABPL ; ou
dans lequel 2 des N protéines de biomarqueur sont le glucagon et l'insuline ; ou
dans lequel 2 des N protéines de biomarqueur sont le glucagon et le PH ; ou
dans lequel 2 des N protéines de biomarqueur sont GIP et FABPL ; ou
dans lequel 2 des N protéines de biomarqueur sont GIP et l'insuline ; ou
dans lequel 2 des N protéines de biomarqueur sont GIP et PH ; ou
dans lequel 2 des N protéines de biomarqueur sont FABPL et l'insuline ; ou
dans lequel 2 des N protéines de biomarqueur sont FABPL et PH ; ou
dans lequel 2 des N protéines de biomarqueur sont l'insuline et PH.

3. Procédé selon la revendication 1 ou 2, dans lequel N est 3, N est 4, N est 5, N est 6, N est 7, facultativement dans lequel la totalité des N protéines de biomarqueur sont choisies parmi FGFP1, TMEDA, glucagon, GIP, FABPL, insuline et PH.

4. Procédé selon l'une quelconque des revendications 1 à 3, dans lequel l'échantillon a été traité, congelé et décongelé après le prélèvement d'échantillon et avant la détection.

5. Procédé selon la revendication 4, dans lequel la détermination est basée sur la comparaison des niveaux détectés des N protéines de biomarqueur à des niveaux de référence, dans lequel les niveaux de référence sont des niveaux moyens des N protéines de biomarqueur présentes dans des échantillons présentant des temps de jeûne de 0,5, 1, 3 ou 12 heures, facultativement dans lequel les niveaux détectés de chacune des N protéines de biomarqueur par rapport aux niveaux de référence indiquent que le temps approximatif qui s'est écoulé entre le repas et le prélèvement d'échantillon était supérieur à 0,5, supérieur à 1, supérieur à 2, supérieur à 3, supérieur à 4, supérieur à 5, supérieur à 6, supérieur à 7, supérieur à 8, supérieur à 9, supérieur à 10, supérieur à 11, ou supérieur à 12 heures.

6. Procédé selon la revendication 4 ou 5, dans lequel la détermination est basée sur un panel de N protéines de biomarqueur présentant une valeur R² d'au moins 0,600, d'au moins 0,650, d'au moins 0,700, d'au moins 0,750, d'au moins 0,800, d'au moins 0,850, d'au moins 0,900 ou d'au moins 0,950.

7. Procédé selon l'une quelconque des revendications 4 à 6, comprenant une réalisation d'une analyse de découverte de biomarqueur protéique, d'une analyse de niveau d'expression de protéine, d'un procédé de diagnostic ou d'un procédé de pronostic sur l'échantillon.

8. Procédé selon l'une quelconque des revendications 1 à 7, comprenant une identification de l'échantillon comme ayant réussi une évaluation de qualité ou échoué à une évaluation de qualité, dans lequel l'identification est basée, au moins en partie, sur les niveaux détectés des N protéines de biomarqueur.

9. Procédé selon la revendication 8, dans lequel l'échantillon est identifié comme réussissant si le temps de jeûne approximatif qui s'est écoulé depuis le repas jusqu'au prélèvement d'échantillon est déterminé comme étant supérieur à 3 heures, supérieur à 4 heures, supérieur à 5 heures, supérieur à 6 heures, supérieur à 7 heures, supérieur à 8 heures, supérieur à 9 heures, supérieur à 10 heures, supérieur à 11 heures, supérieur à 12 heures, supérieur à 13 heures, supérieur à 14 heures, supérieur à 15 heures, supérieur à 16 heures, supérieur à 17 heures, supérieur à 18 heures, supérieur à 19 heures, supérieur à 20 heures, supérieur à 21 heures, supérieur à 22 heures, supérieur à 23 heures, ou supérieur à 24 heures.

10. Procédé selon l'une quelconque des revendications 1 à 9, dans lequel la détection comprend une réalisation d'une spectrométrie de masse, d'un dosage à base d'aptamère et/ou d'un dosage à base d'anticorps.

11. Procédé selon l'une quelconque des revendications précédentes, dans lequel le procédé comprend une mise en contact de protéines de biomarqueur de l'échantillon provenant du sujet avec un ensemble de réactifs de capture, dans lequel chaque réactif de capture de l'ensemble de réactifs de capture se lie de manière spécifique à une protéine de biomarqueur détectée, facultativement dans lequel chaque réactif de capture se lie de manière spécifique à une protéine de biomarqueur différente détectée.

12. Procédé selon la revendication 11, dans lequel chaque réactif de capture est un anticorps ou un aptamère.

13. Procédé selon la revendication 12, dans lequel chaque réactif de capture est un aptamère.

14. Procédé selon la revendication 13, dans lequel au moins un aptamère est un aptamère à vitesse de dissociation lente, facultativement, dans lequel chaque aptamère à vitesse de dissociation lente se lie à sa protéine cible avec une vitesse de dissociation (t_{1/2}) > 30 minutes, ≥ 60 minutes, ≥ 90 minutes, ≥ 120 minutes, ≥ 150 minutes, ≥ 180 minutes, ≥ 210 minutes ou ≥ 240 minutes, et/ou facultativement dans lequel au moins un aptamère à vitesse de dissociation lente comprend au moins 1, au moins 2, au moins 3, au moins 4, au moins 5, au moins 6, au moins 7, au moins 8, au moins 9 ou au moins 10 nucléotides avec des modifications.
